(19) Europäisches Patentamt
European Patent Office
Office européen des brevets

(11) **EP 4 343 775 A1**

(12) **EUROPEAN PATENT APPLICATION**

(43) Date of publication:
**27.03.2024 Bulletin 2024/13**

(21) Application number: **22306403.1**

(22) Date of filing: **22.09.2022**

(51) International Patent Classification (IPC):
*G16H 20/00* (2018.01)   *G16H 50/20* (2018.01)
*G16H 50/50* (2018.01)   *G16H 50/70* (2018.01)

(52) Cooperative Patent Classification (CPC):
**G16H 50/20; G16H 10/40; G16H 20/10;**
**G16H 20/40; G16H 50/30; G16H 50/70**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **SurgeCare**
**67400 Illkirch-Graffenstaden (FR)**

(72) Inventor: **BELLAN, Grégoire**
**75116 PARIS (FR)**

(74) Representative: **Lavoix**
**2, place d'Estienne d'Orves**
**75441 Paris Cedex 09 (FR)**

(54) **A METHOD FOR DETERMINING A MEDICAL OUTCOME FOR AN INDIVIDUAL, RELATED ELECTRONIC SYSTEM AND COMPUTER PROGRAM**

(57)     A method for determining a medical outcome for an individual, comprises:
- obtaining values of a plurality of features, said plurality including omic biological and clinical features;
- computing a medical score related to the medical outcome for the individual based on the plurality of features using a model obtained via a machine learning technique; and
- providing an assessment of the medical outcome based on the computed score.

The machine learning model is trained using a bootstrap procedure and includes weights for a set of selected biological and clinical or demographic features.

During the machine learning, initial weights are computed for the features using a statistical learning technique.

A feature is selected when its occurrence frequency of significant weights among the initial weights is greater than a frequency threshold that is computed to minimize a ratio depending on the number of selected artificial feature(s) divided by the number of selected features.

FIG.1

EP 4 343 775 A1

**Description**

**FIELD OF THE INVENTION**

The present invention relates to a method for determining a medical outcome for an individual.

The invention also relates to an electronic system comprising a processor and memory containing instructions, which when executed by the processor, direct the processor to perform such a method; and also to a computer program comprising software instructions which, when executed by a processor, carry out such a method.

The present invention relates to predicting medical outcome, more specifically, using a machine learning model to predict medical outcomes from clinical and multi-omics data. The medical outcomes are typically surgical outcomes, such as post-operative infections and surgical site complications; postoperative complications, such as cognitive disorders, surgical site infection; clinical events, such as pre-term, heart failure, stroke, onset of labor, renal failure; drug efficiency, such as chemotherapy, Immunotherapy using the recession of the illness as the outcome, any other drug; side effects and allergies, such as immunotherapy, contrast agent, etc.; cancer monitoring, such as tumor evolution, illness progression, relapse; surgical recovery, such as length of stay, infections, hemorrhagic, depression, well-being, cost in care; auto-immune and chronic diseases diagnosis, such as isolating biomarkers highly correlated to auto-immune or chronic diseases to strengthen the diagnosis; and diet efficiency, impact of food and supplements.

**BACKGROUND OF THE INVENTION**

Over 300 million operations are performed annually worldwide, a number that is expected to increase. Surgical complications including infection, protracted pain, functional impairment, and end-organ damage occur in 10-60% of surgeries, causing personal suffering, longer hospital stays, readmissions, and significant socioeconomic burden. After major abdominal operations, surgical site complications (SSCs), including superficial or deep wound infections, organ space infections, anastomotic leaks, fascial dehiscence, and incisional hernias, are some of the most devastating, costly, and common surgical complications occurring in up to 25% of patients. (See e.g., Healy MA et al. JAMA Surg 2016; 151(9):823-30).

The method of the invention initially arises from the observation that existing risk prediction tools are based on clinical parameters and are insufficient to estimate an individual patient's risk for SSCs (See e.g., Eamer G, et al. Am J Surg 2018; 216(3):585-594; and Cohen ME et al. J Am Coll Surg 2017; 224(5):787-795 e1). The accurate prediction of SSC risk for individual patients is critically important to guide high-quality surgical decision making, including optimizing preoperative interventions and timing of surgery. Existing risk prediction tools are based on clinical parameters and are insufficient to estimate an individual patient's risk for SSCs. As such, there is a need in the field for a robust tool to predict SSCs with more accuracy.

Surgery is associated with significant tissue trauma, triggering a programmed inflammatory response that engages the innate and adaptive branches of the immune system. Within hours of surgical incision, a highly diverse network of innate immune cells (including monocytes, neutrophils and their subsets) is activated in response to circulating DAMPs (damage-associated molecular patterns) and inflammatory cytokines (e.g., HMGB1, TNF$\alpha$, and IL-1$\beta$). Following the early innate immune response to surgery, a compensatory, anti-inflammatory adaptive immune response has been traditionally described. However, recent transcriptomic and mass cytometry analysis suggest that adaptive immune responses are mobilized jointly with innate immune responses and coincide with the activation of specialized immunosuppressive immune cell subsets, such as myeloid-derived suppressor cells (MDSCs). In the context of uncomplicated surgical recovery, innate and adaptive responses synergize to orchestrate pro- and anti-inflammatory (pro-resolving) processes required for pathogen defense tissue remodeling and the resolution of pain and inflammation after injury. (See e.g.,Stoecklein VM et al. J Leukoc Biol 2012; Gaudilliere B et al. Sci Trans! Med 2014; 6(255):255ra131).

Complications including infections, wound dehiscence, and eventually end-organ damage arise as pro-inflammatory and immune-suppressive responses tilt out of balance. A detailed characterization of immunological mechanisms that differ between patients with and without surgical complications is thus a highly promising approach for identifying pre-and post-operative biological events that contribute to and precede surgical complications. Prior attempts to detect biological markers predicting the risk for SSCs focused on secreted humoral factors, surface marker expression on select immune cells or transcriptional analysis of pooled circulating leukocytes. However, detected associations were insufficient to accurately predict the risk of SSCs for individual patients.

A major impediment has been the lack of high-content, functional assays that can characterize the complex, multicellular inflammatory response to surgery with single-cell resolution. In addition, analytical tools that can integrate the single-cell immunological data with other 'omics and clinical data to predict the development of SSC are lacking. Thus, there is a need for improved measures for the diagnosis, prognosis, treatment, management, and therapeutic development of SSC after surgery.

High-throughput omics assays including metabolomics, proteomic and cytometric immunoassay data can potentially capture complex mechanism of diseases and biological processes by providing thousands of measurements systematically obtained on each biological sample.

The analysis of mass cytometry immunoassay as well as other omics assays typically has two related goals analyzed by dichotomous approaches. The first goal is to predict the outcome of interest and identify biomarkers that are the best set of predictors of the considered outcome; the second goal is to identify potential pathways implicated in the disease offering better understanding into the underlying biology. The first goal is addressed by deploying machine learning methods and fitting a prediction model that selects typically a handful of most informative biomarkers among thousands of measurements. The second goal is usually addressed by performing univariate analysis of each measurement to determine the significance of that measurement with respect to the outcome by evaluating its p-value that is then adjusted for multiple hypothesis testing.

In the context of machine learning, omics data - characterized by a high number of features $p$ and a much smaller number of samples $n$ - fall in the scenario for which $p >> n$. The gold-standard machine learning methodology for this scenario consists of the usage of regularized regression or classification methods, and specifically sparse linear models, such as the Lasso; *(See e.g.,* Tibshirani, Robert. "Regression shrinkage and selection via the lasso." Journal of the Royal Statistical Society: Series B (Methodological) 58.1 (1996): 267-288) and Elastic Net. *(See e.g.,* Zou, Hui, and Trevor Hastie. "Regularization and variable selection via the elastic net." Journal of the royal statistical society: series B (statistical methodology) 67.2 (2005): 301-320) Consider for instance the following linear model, given by:

Equation (1)

$$Y = X\beta + \epsilon$$

where $X = (X_1, \ldots, X_n) \in \mathbb{R}^{n \times p}$ and $Y = (Y_1, \ldots, Y_n) \in \mathbb{R}^n$ are respectively the input and the response variables; $\epsilon = (\epsilon_1, \ldots, \epsilon_n) \in \mathbb{R}^n$ is the random noise with independent, identically distributed components. $\beta = (\beta_1, \ldots, \beta_p) \in \mathbb{R}^p$ are the coefficients associated to each feature, that we need to learn. Sparse linear models add a regularization of the model coefficients $\beta$, which allows for balancing the bias-variance tradeoff and prevents overfitting of models. The Lasso and the Elastic Net use L1-regularization in the model, inducing sparsity in the fit of the coefficients $\beta$. In the optimal fit of such models, we end up determining a subset $S = \{\beta_k, \beta_k \neq 0\}$ where many of the coefficients $\beta$ become zeros, resulting in only a subset of features playing a role in the model.

Instability is an inherent problem in feature selection of machine learning model. Since the learning phase of the model is performed on a finite data sample, any perturbation in data may yield a somewhat different set of selected variables. In settings where the performance is evaluated via cross-validation, this implies that the Lasso yields a somewhat different set of chosen biomarkers making any biological interpretation of the result impossible. Consistent feature selection in Lasso is challenging as it is achieved only under restrictive conditions. Most sparse techniques such as the Lasso cannot provide a quantification of how far the chosen model is from the correct one, nor quantify the variability of chosen features.

Another major limitation of existing methods is the difficulty to integrate different sources of biological information. Most machine learning algorithms use input data agnostically in the learning process of the models. The main challenge lies in the integration of multiple sources of data with their differences in modalities, size and signal-to-noise ratio in the learning process. In the learning process, current approach are typically limited with biased assessment of the contribution of individual sources of data when juxtaposed as a unique dataset. Finally, it is key to use identified informative features from different layers together to optimize the predictive power of such algorithms. Most methods, when ensembling different results from individual data sources also lack the capacity to assess individual interactions between features that are key to model biological mechanisms at play.

In this background, the article "Integrated single-cell and plasma proteomic modeling to predict surgical site complications, a prospective cohort study" from Kristen K. Rumer et al. (Ann Surg. 2022;275(3):582-590) aims to determine whether single-cell and plasma proteomic elements of the host's immune response to surgery accurately identifies patients who develop SSC after abdominal surgery. A secondary objective was to determine whether patient-specific immune states before surgery differentiate patients with or without an SSC.

This article discloses an integrated approach combining the functional analysis of immune cell subsets using mass cytometry with the highly multiplexed assessment of inflammatory plasma proteins to quantify the dynamic changes of over 2,388 single-cell and plasma proteomic events in patients before and after major abdominal surgery. The primary aim of the study presented in this article was to identify elements of the immune response to surgery that differentiate patients with or without an SSC with sufficient predictive performance. It was also examined whether patient-specific immune states before surgery could differentiate these patient groups.

However, the results obtained with this approach are of variable quality, and depend notably on the structure of the input data.

## SUMMARY OF THE INVENTION

An object of the invention is therefore to provide a more efficient method for determining a medical outcome for an individual.

For this purpose, the subject-matter of the invention is a method for determining a medical outcome for an individual, comprising:

- obtaining or having obtained values of a plurality of features, wherein the plurality of features includes omic biological features and clinical features;
- computing a medical score related to the medical outcome for the individual based on the plurality of features using a model obtained via a machine learning technique; and
- providing an assessment of the individual's medical outcome based on the computed medical score;

wherein the machine learning model is trained using a bootstrap procedure on a plurality of individual data layers, wherein each data layer represents one type of data from the plurality of features and at least one artificial feature; wherein the model includes weights for a set of selected biological and clinical or demographic features; during the machine learning and for each data layer, for every repetition of the bootstrap, initial weights are computed for the plurality of features and/or the at least one artificial feature associated with that data layer using an initial statistical learning technique, and at least one feature is selected; wherein a feature is selected when an occurrence frequency associated to said feature of significant weights among the computed initial weights is greater than a frequency threshold, the frequency threshold being computed according to the occurrence frequencies obtained for the artificial features; and wherein the frequency threshold is computed to minimize a ratio depending on the number of selected artificial feature(s) divided by the number of selected features.

The method according to the invention therefore involves a False Discovery Rate metric control for the features selection, the number of selected artificial feature(s) corresponding to False Positive(s) and the number of selected non-artificial feature(s) corresponding to True Positive(s). Thus, with the method according to the invention, the features selection is improved, leading to better results for determining the medical outcome.

According to other advantageous aspects of the invention, the method comprises one or several of the following features, taken individually or according to any technically possible combination:

- the initial weights are further computed for a plurality of values of a hyperparameter, and the hyperparameter is a parameter whose value is used to control the learning process;

  the hyperparameter being preferably a regularization coefficient used according to a respective mathematical norm in the context of a sparse initial technique;
  the mathematical norm being further preferably a p-norm, with p being an integer;

- for each feature, a unitary occurrence frequency is calculated for each hyperparameter value and is equal to a number of the significant weights related to said feature for the successive bootstrap repetitions divided by the number bootstrap repetitions;
  the occurrence frequency being preferably equal to the highest unitary occurrence frequency among the unitary occurrence frequencies calculated for the plurality of hyperparameter values;
- the frequency threshold computing includes:

  + calculating successively several ratios with respect to successive threshold values, each ratio depending on the number of selected artificial feature(s) with respect to a respective threshold value divided by the number of selected features with respect to said threshold value,
  + the computed frequency threshold being equal the threshold value corresponding to the minimum calculated ratio among the calculated ratios for the successive threshold values;

  each ratio ($FDR_\tau$) being preferably calculated according to the following equation:

$$FDR_\tau = \frac{FP_\tau + 1}{FP_\tau + TP_\tau}$$

where $FP_\tau$ is the number of selected artificial feature(s) and $TP_\tau$ is the number of selected non-artificial feature(s) for a given threshold value $\tau$, $FP_\tau + TP_\tau$ being therefore equal to the number of selected features;

- the initial statistical learning technique is selected from the group consisting in: a regression technique, a classification technique and an ensembling technique;

  the initial statistical learning technique being preferably selected from a sparse technique and a non-sparse technique;
  the sparse technique being further preferably selected from a Lasso technique and an Elastic Net technique;
  the ensembling technique being further preferably selected from a Random Forest technique or a XGBoost technique;

- the significant weights are non-zero weights, when the initial statistical learning technique is a sparse regression technique; and
  the significant weights are weights above a predefined weight threshold, when the initial statistical learning technique is a non-sparse regression technique;
- the significant weights are weights above a computed weight threshold, and the weight threshold computation includes:

  + calculating, for each hyperparameter value, a ratio depending on the number of artificial feature(s) with an associated weight above a unitary weight threshold associated to hyperparameter value divided by the number of features with an associated weight above said unitary weight threshold, each unitary weight threshold being strictly positive and predefined,
  + the computed weight threshold being equal to the unitary weight threshold corresponding to the minimum calculated ratio among the calculated ratios for the plurality of hyperparameter values;

  each ratio ($FDR_\lambda$) being preferably calculated according to the following equation:

$$FDR_\lambda = \frac{\#\{|\beta_i| > \beta_\lambda, \text{for i of artificial features}\} + 1}{\#\{|\beta_i| > \beta_\lambda, \text{for i of all features}\}}$$

  where # represents the number of respective features verifying the condition $|\beta_i| > \beta_\lambda$; i is an index associated to each feature; $\beta_i$ represents the weight for the feature of index i; and $\beta_\lambda$ represents the unitary weight threshold associated to hyperparameter value $\lambda$;

- the hyperparameter is an upper bound of the coefficient of the L1-norm of the initial weights when the initial statistical learning technique is the Lasso technique, wherein the L1-norm refers to the sum of all absolute values of the initial weights; and
  the hyperparameter being an upper bound of the coefficient of the to both the L1-norm sum of the initial weights and the L2-norm sum of the initial weights when the initial statistical learning technique is the Elastic Net technique, the L1-norm refers to the sum of all absolute values of the initial weights, and L2-norm refers to the square root of the sum of all squared values of the initial weights;
- omic biological features comprise at least one feature selected from the group consisting of a genomic feature, a transcriptomic feature, a proteomic feature, a cytomic feature, and a metabolomic feature;

  the plurality of features preferably further including clinical and/or demographic features;
  each type being further preferably selected from the group consisting of genomic, transcriptomic, proteomic, cytomic, metabolomic, clinical and demographic data;

- each data layer comprises data for a population of individuals; each feature includes feature values for individuals in the population of individuals; and

for a respective data layer, each artificial feature is obtained from a non-artificial feature among the plurality of features, via a mathematical operation performed on the feature values of the non-artificial feature;

the mathematical operation being preferably chosen among the group consisting of: a permutation, a sampling with replacement, a sampling without replacement, a combination, a Knockoff sampling and an inference;

obtaining artificial features being preferably done once during the bootstrap procedure;

- during the machine learning, the method further comprises, before obtaining artificial features, generating additional values of the plurality of non-artificial features based on the obtained values and using a data augmentation technique;

the artificial features being then obtained according to both the obtained values and the generated additional values;

the data augmentation technique being preferably chosen among a non-synthetic technique and a synthetic technique;

the data augmentation technique being further preferably chosen among the group consisting of: SMOTE technique, ADASYN technique and SVMSMOTE technique;

for a given non-artificial feature, the less values have been obtained, the more additional values being preferably generated;

- during the machine learning, the weights of the model are further computed using a final statistical learning technique on the data associated to the set of selected features;

the final statistical learning technique being preferably selected from the group consisting in: a regression technique, a classification technique and an ensembling technique;

the final statistical learning technique being preferably selected from a sparse technique and a non-sparse technique;

the sparse technique being further preferably selected from a Lasso technique and an Elastic Net technique, or else from a non-linear technique, such as the Generalized Additive Model technique;

the ensembling technique being further preferably selected from a Random Forest technique or a XGBoost technique;

- during a usage phase subsequent to the machine learning, the medical score is computed according to the measured values of the individual for the set of selected features;

the medical score being preferably a probability calculated according to a weighted sum of the measured values multiplied by the respective weights for the set of selected features, when the final statistical learning technique is the classification technique; the medical score being further preferably calculated according to the following equation:

$$P = \frac{Odd}{1 + Odd}$$

where P represents the medical score, and
*Odd* is a term depending on the weighted sum; *Odd* being for example an exponential of the weighted sum;

the medical score being preferably a term depending on a weighted sum of the measured values multiplied by the respective weights for the set of selected features, when the final statistical learning technique is the regression technique; the medical score being further preferably equal to an exponential of the weighted sum;

- the medical outcome is selected from the group consisting of postoperative complications, clinical event, drug efficiency or patient's responsiveness to treatment, drug side effects and allergies, surgical recovery, auto-immune and chronic diseases diagnosis, diet efficiency, impact of food and supplements, and infection complication;
- obtaining or having obtained values of a plurality of features comprises:

+ obtaining or having obtained a sample for analysis from the individual subject to surgery; and
+ measuring or having measured the values of a plurality of omic biological and clinical features;

- the plurality of features further includes demographic features;

- the number bootstrap repetitions is between 50 and 100,000;
- the plurality of hyperparameter values is between 0.5 and 100 for the Lasso technique or the Elastic Net technique;

The method may further comprise defining a suitable treatment and/or treating the individual in accordance with the assessment of an individual's medical outcome.

The subject-matter of the invention is also an electronic system comprising a processor and memory containing instructions, which when executed by the processor, direct the processor to perform the method as defined above.

The invention also concerns a computer program comprising software instructions which, when executed by a processor, carry out the method as defined above.

According to other advantageous aspects of the invention, the method comprises one or several of the following features, taken individually or according to any technically possible combination:

when the medical outcome is a surgical outcome, the method further comprises treating the individual before surgery in accordance with the assessment of an individual's risk for developing a surgical site complication;

when the medical outcome is a surgical outcome, the method further comprises treating the individual after surgery in accordance with the assessment of an individual's risk for developing a surgical site complication.

## BRIEF DESCRIPTION OF THE DRAWINGS

The invention will be better understood upon reading of the following description, which is given solely by way of example and with reference to the appended drawings, wherein:

- Figure 1 illustrates an exemplary method for determining a medical outcome for an individual using a Multi-Omic Bootstrap (MOB) machine learning algorithm that integrates multi-omic biological (e.g. single cell immune responses and plasma proteomic data) and clinical data;
- Figure 2 illustrates an exemplary methodology for the MOB machine learning algorithm that integrates biological and clinical data for the prediction of medical outcomes;
- Figure 3 illustrates a block diagram of components of a processing system in a computing device that can be used to compute a medical score related to the medical outcome;
- Figure 4 illustrates a network diagram of a distributed system to generate the medical score;
- Figure 5 shows results obtained with the invention, in particular for features selection, according to a first example of the medical outcome;
- Figure 6, and respectively Figure 7, are views similar to that of Figure 5, according to a second example, and respectively a third example, of the medical outcome.

## DETAILED DESCRIPTION

### Definitions

Most of the words used in this specification have the meaning that would be attributed to those words by one skilled in the art. Words specifically defined in the specification have the meaning provided in the context of the present teachings as a whole, and as are typically understood by those skilled in the art. In the event that a conflict arises between an artunderstood definition of a word or phrase and a definition of the word or phrase as specifically taught in this specification, the specification shall control.

All publications, patents, and patent applications mentioned in this specification are herein incorporated by reference to the same extent as if each individual publication, patent, or patent application was specifically and individually indicated to be incorporated by reference.

It must be noted that, as used in the specification and the appended claims, the singular forms "a," "an," and "the" include plural referents unless the context clearly dictates otherwise.

The terms "subject," "individual," and "patient" are used interchangeably herein to refer to a vertebrate, preferably a mammal, more preferably a human. Mammalian species that provide samples for analysis include canines; felines; equines; bovines; ovines; *etc.* and primates, particularly humans. Animal models, particularly small mammals, e.g. murine, lagomorpha, *etc.* can be used for experimental investigations. The methods of the invention can be applied for veterinary purposes. The terms "biomarker," "biomarkers," "marker", "features", or "markers" for the purposes of the invention refer to, without limitation, proteins together with their related metabolites, mutations, variants, polymorphisms, phosphorylation, modifications, fragments, subunits, degradation products, elements, and other analytes or sample-derived measures. Markers can include expression levels of an intracellular protein or extracellular protein. Markers can also include combinations of any one or more of the foregoing measurements, including temporal trends and differences.

Broadly used, a marker can also refer to an immune cell subset.

As use herein, the term "omic" or "-omic" data refers to data generated to quantify pools of biological molecules, or processes that translate into the structure, function, and dynamics of an organism or organisms. Examples of omic data include (but are not limited to) genomic, transcriptomic, proteomic, metabolomic, cytomic data, among others.

As use herein the term "cytomic" data refers to an omic data generated using a technology or analytical platform that allows quantifying biological molecules or processes at the single-cell level. Examples of cytomic data include (but are not limited to) data generated using flow cytometry, mass cytometry, single-cell RNA sequencing, cell imaging technologies, among others. Cytomic data define the molecular phenotype of single cells.

The term "inflammatory" response is the development of a humoral (antibody mediated) and/or a cellular response, which cellular response may be mediated by innate immune cells (such as neutrophils or monocytes) or by antigen-specific T cells or their secretion products. An "immunogen" is capable of inducing an immunological response against itself on administration to a mammal or due to autoimmune disease.

To "analyze" includes determining a set of values associated with a sample by measurement of a marker (such as, e.g., presence or absence of a marker or constituent expression levels) in the sample and comparing the measurement against measurement in a sample or set of samples from the same subject or other control subject(s). The markers of the present teachings can be analyzed by any of various conventional methods known in the art. To "analyze" can include performing a statistical analysis, e.g. normalization of data, determination of statistical significance, determination of statistical correlations, clustering algorithms, and the like.

A "sample" in the context of the present teachings refers to any biological sample that is isolated from a subject, generally a blood or plasma sample, which may comprise circulating immune cells. A sample can include, without limitation, an aliquot of body fluid, plasma, serum, whole blood, PBMC (white blood cells or leucocytes), tissue biopsies, dissociated cells from a tissue sample, a urine sample, a saliva sample, synovial fluid, lymphatic fluid, ascites fluid, and interstitial or extracellular fluid. "Blood sample" can refer to whole blood or a fraction thereof, including blood cells, plasma, serum, white blood cells or leucocytes. Samples can be obtained from a subject by means including but not limited to venipuncture, biopsy, needle aspirate, lavage, scraping, surgical incision, or intervention or other means known in the art.

A "dataset" is a set of numerical values resulting from evaluation of a sample (or population of samples) under a desired condition. The values of the dataset can be obtained, for example, by experimentally obtaining measures from a sample and constructing a dataset from these measurements; or alternatively, by obtaining a dataset from a service provider such as a laboratory, or from a database or a server on which the dataset has been stored. Similarly, the term "obtaining a dataset associated with a sample" encompasses obtaining a set of data determined from at least one sample. Obtaining a dataset encompasses obtaining a sample, and processing the sample to experimentally determine the data, e.g., via measuring antibody binding, or other methods of quantitating a signaling response. The phrase also encompasses receiving a set of data, e.g., from a third party that has processed the sample to experimentally determine the dataset.

"Measuring" or "measurement" in the context of the present teachings refers to determining the presence, absence, quantity, amount, or effective amount of a substance in a clinical or subject-derived sample, including the presence, absence, or concentration levels of such substances, and/or evaluating the values or categorization of a subject's clinical parameters based on a control, e.g. baseline levels of the marker.

Classification can be made according to predictive modeling methods that set a threshold for determining the probability that a sample belongs to a given class. The probability preferably is at least 50%, or at least 60% or at least 70% or at least 80% or higher. Classifications also can be made by determining whether a comparison between an obtained dataset and a reference dataset yields a statistically significant difference. If so, then the sample from which the dataset was obtained is classified as not belonging to the reference dataset class. Conversely, if such a comparison is not statistically significantly different from the reference dataset, then the sample from which the dataset was obtained is classified as belonging to the reference dataset class.

The predictive ability of a model can be evaluated according to its ability to provide a quality metric, e.g. Area Under the Curve (AUC) or accuracy, of a particular value, or range of values. In some embodiments, a desired quality threshold is a predictive model that will classify a sample with an accuracy of at least about 0.7, at least about 0.75, at least about 0.8, at least about 0.85, at least about 0.9, at least about 0.95, or higher. As an alternative measure, a desired quality threshold can refer to a predictive model that will classify a sample with an AUC of at least about 0.7, at least about 0.75, at least about 0.8, at least about 0.85, at least about 0.9, or higher.

As is known in the art, the relative sensitivity and specificity of a predictive model can be "tuned" to favor either the selectivity metric or the sensitivity metric, where the two metrics have an inverse relationship. The limits in a model as described above can be adjusted to provide a selected sensitivity or specificity level, depending on the particular requirements of the test being performed. One or both of sensitivity and specificity can be at least about at least about 0.7, at least about 0.75, at least about 0.8, at least about 0.85, at least about 0.9, or higher.

As used herein, the term "theranosis" refers to the use of results obtained from a prognostic or diagnostic method to direct the selection of, maintenance of, or changes to a therapeutic regimen, including but not limited to the choice of one or more therapeutic agents, changes in dose level, changes in dose schedule, changes in mode of administration, and changes in formulation. Diagnostic methods used to inform a theranosis can include any that provides information on the state of a disease, condition, or symptom.

The terms "therapeutic agent", "therapeutic capable agent" or "treatment agent" are used interchangeably and refer to a molecule, compound or any non-pharmacological regimen that confers some beneficial effect upon administration to a subject. The beneficial effect includes enablement of diagnostic determinations; amelioration of a disease, symptom, disorder, or pathological condition; reducing or preventing the onset of a disease, symptom, disorder or condition; and generally counteracting a disease, symptom, disorder or pathological condition.

As used herein, "treatment" or "treating," or "palliating" or "ameliorating" are used interchangeably. These terms refer to an approach for obtaining beneficial or desired results including but not limited to a therapeutic benefit and/or a prophylactic benefit. By therapeutic benefit is meant any therapeutically relevant improvement in or effect on one or more diseases, conditions, or symptoms under treatment. For prophylactic benefit, the compositions may be administered to a subject at risk of developing a particular disease, condition, or symptom, or to a subject reporting one or more of the physiological symptoms of a disease, even though the disease, condition, or symptom may not have yet been manifested.

The term "effective amount" or "therapeutically effective amount" refers to the amount of an agent that is sufficient to effect beneficial or desired results. The therapeutically effective amount will vary depending upon the subject and disease condition being treated, the weight and age of the subject, the severity of the disease condition, the manner of administration and the like, which can readily be determined by one of ordinary skill in the art. The term also applies to a dose that will provide an image for detection by any one of the imaging methods described herein. The specific dose will vary depending on the particular agent chosen, the dosing regimen to be followed, whether it is administered in combination with other compounds, timing of administration, the tissue to be imaged, and the physical delivery system in which it is carried.

"Suitable conditions" shall have a meaning dependent on the context in which this term is used. That is, when used in connection with an antibody, the term shall mean conditions that permit an antibody to bind to its corresponding antigen. When used in connection with contacting an agent to a cell, this term shall mean conditions that permit an agent capable of doing so to enter a cell and perform its intended function. In one embodiment, the term "suitable conditions" as used herein means physiological conditions.

The term "antibody" includes full length antibodies and antibody fragments, and can refer to a natural antibody from any organism, an engineered antibody, or an antibody generated recombinantly for experimental, therapeutic, or other purposes as further defined below. Examples of antibody fragments, as are known in the art, such as Fab, Fab', F(ab')2, Fv, scFv, or other antigen-binding subsequences of antibodies, either produced by the modification of whole antibodies or those synthesized de novo using recombinant DNA technologies. The term "antibody" comprises monoclonal and polyclonal antibodies. Antibodies can be antagonists, agonists, neutralizing, inhibitory, or stimulatory. They can be humanized, glycosylated, bound to solid supports, and possess other variations.

## Method for determining a medical outcome

The method for determining a medical outcome as described herein is generally applicable to the determination of any medical outcome in an individual, the method being able to isolate the most predictive features for the observed medical outcome.

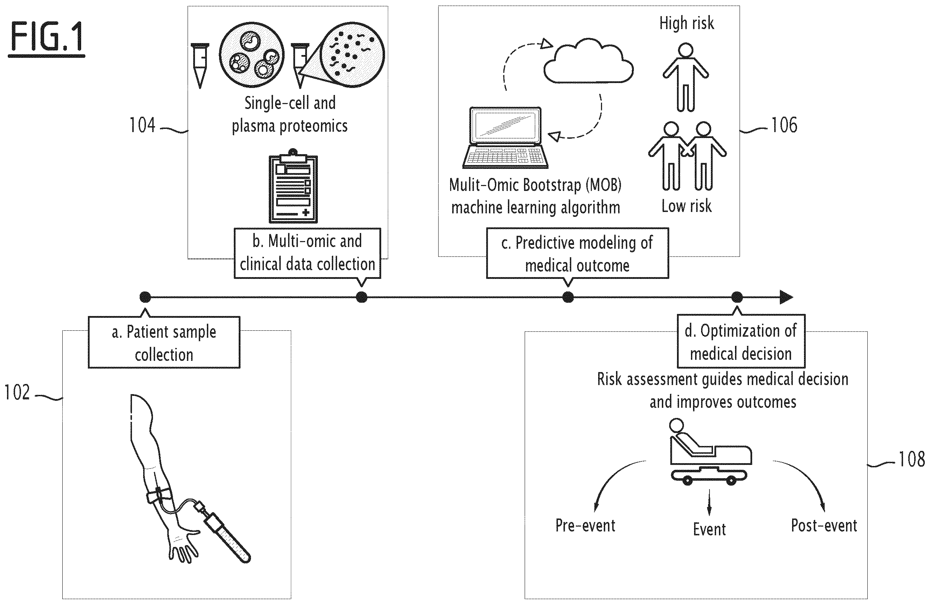

In particular, methods are provided for the prediction, classification, diagnosis, and/or theranosis, of a medical outcome for an individual based on the integration of multi-omic biological, and clinical data (and optionally demographic data) using a machine learning model (e.g., Figure 1).

According to the method of the invention, a predictive model of a patient's probability to have a medical outcome is generated wherein the predictive model is obtained by quantitating specific biological, and clinical features, or biological, clinical and demographic features, in a population of individuals.

The method can be used to perform predictions of any medical outcome in an individual. In some embodiments, the medical outcomes is selected from the group consisting of postoperative complications, clinical event, drug efficiency or patient's responsiveness to treatment, drug side effects and allergies, surgical recovery, autoimmune and chronic diseases diagnosis, diet efficiency, impact of food and supplements, and infection complication.

In some embodiments, the method enables predicting postoperative complications such as onset of cognitive disorders, surgical site complications (SSCs) including surgical site infections, organ space infections, anastomotic leaks, fascial dehiscence, and incisional hernia surgical site infections.

In some embodiments, the method enables predicting a clinical event, such as preterm delivery or labor onset in pregnant woman, heart failure, stroke, and renal failure.

In some embodiments, the method enables predicting drug efficiency in a patient or patient's responsiveness to treatment, such as chemotherapy, immunotherapy, or any other drug, or predicting patient's responsiveness to alternative medicine efficiency. In such cases the recession of the illness is used as the outcome to predict.

In some embodiments, the method enables predicting occurrence of side effects and allergies further to drug administration, e.g. further to administration of an immunotherapeutic drug, a contrast agent, etc.

In some embodiments, the method enables cancer monitoring, by predicting for instance tumor evolution, illness progression, or relapse.

In some embodiments, the method enables predicting surgical recovery in a patient, such as length of stay in hospital, infection, hemorrhagic, depression, well-being, costs in care.

In some embodiments, the method enables diagnosing auto-immune and chronic diseases by isolating biomarkers highly correlated to auto-immune or chronic diseases to strengthen the diagnosis.

In some embodiments, the method enables predicting infection complication such as development of sepsis or septic shock further to bacterial or fungal infection, or development of severe form of disease further to viral infection such as development of severe COVID-19 in patients infected with SARS-COV2.

In some embodiments, the method enables predicting patient's responsiveness to treatment. Based on perturbation in omics collected from drugs databases, the method enables for making drug recommendations based on the individual's immune profile as determined by the computed medical score related to the medical outcome for the individual.

The outcomes are either categorical (such as onset or no onset of an outcome; e.g. of a surgical or infection complication; or categorization of the risk towards a mild, moderate or severe form of a disease), or continuous (e.g. duration of hospitalization, time before labor onset).

As exemplary embodiments, the method enables prediction of:

- Labor onset based on a predictive Model that consists of a linear regression of selected features that are listed in Table 1 of the example section;
- Diagnosis of COVID19 severity based on a predictive Model that integrates selected features that are listed in Table 3 of the example section;
- Surgical site infection based on a predictive Model that integrates selected features that are listed in Table 5 of the example section.

After the determination of the medical outcome for the individuals, in some embodiments, the method further comprises defining a suitable treatment or patient care management and/or treating the individual in accordance with the assessment of an individual's medical outcome. In particular, a monitoring of the individuals may be implemented during their treatment based on biomarkers correlated with the medical outcome, or treatment recommendations can be given to new patients based on their omics' profile, with the aim of optimizing the outcome of the treatment.

The invention also relates to a therapeutic drug for use for the treatment of a patient whose medical outcome was determined by the method of the invention.

In some embodiments, when the medical outcome is a surgical outcome, the method further comprises treating the individual before and/or after surgery in accordance with the assessment of an individual's risk for developing a surgical site complication or cognitive disorder. In particular, the medical score related to the medical outcome provides objective information for the surgical care team at several moment in the perioperative care pathway, in order to implement pre-operative, intra-operative and/or post-operative measures. Preoperative measures include implementation of Enhanced Recovery After Surgery (ERAS) protocol, implementation of prehabilitation protocols, optimization of the timing of surgery, and/or optimization of preoperative therapeutic regimen (timing of chemotherapeutic drugs or immunomodulators). Intra-operative measures include optimization of surgical approach (e.g. decision to perform a diverting ileostomy), decision to perform minimally invasive surgery vs. open surgery, and/or optimization of peri-operative antibiotic regimen (in particular when the medical outcome is a surgical site infection). Postoperative measures include decision to utilize advance wound care technique (e.g. wound vac), patient hospital discharge planning, and/or need for post-discharge advanced wound care or rehabilitation. Appropriate care can reduce the rate of SSCs, length of hospital stays, and/or the rate of readmission for patients following surgery.

In some embodiments, when the medical outcome is prediction of labor onset, the method further comprises increasing frequency of clinic visits and pregnancy monitoring for patients at risk of preterm labor, taking clinical decision for medical therapy (progesterone therapy) or surgical interventions (cerclage), and/or informing post-birth clinical decision making (e.g. hospital admission of newborn).

In some embodiments, when the medical outcome is prediction and/or diagnosis of COVID19 severity score, the method further comprises decision to admit patient to the hospital, decision to admit patient to intensive care unit, decision to administer therapeutic treatment such as corticosteroids (e.g. Dexamethasone), immunoglobulins, and/or antiviral drugs (e.g. paxlovid, nirmatrelvir).

**Methods for generating multi-omic biological data**

The method for determining a medical outcome for an individual uses at least one omic feature, preferably in combination with the clinical and demographic data of a population of individuals, to generate a predictive model. Various embodiments utilize a machine learning model to integrate the various clinical, demographic data and/or omic features to generate the predictive model.

The method for determining a medical outcome for an individual thus comprises obtaining or having obtained values of a plurality of features for the individual, wherein the plurality of features includes omic biological features, and demographic and clinical features; computing a medical score related to the medical outcome for the individual based on the plurality of features using a (predictive) model obtained via a machine learning technique; and providing an assessment of the individual's medical outcome based on the computed medical score.

In many embodiments, the method for generating a predictive model of a medical outcome relies on the multi-omic analysis of biological samples obtained from a population of individuals to obtain a determination of changes e.g., in immune cell subset frequencies and signaling activities, and in plasma proteins. The population of individuals includes individuals in a clinical situation relevant for the medical outcome, for instance patients requiring surgery if the medical outcome is postoperative complications; pregnant women if the outcome labor onset; etc.

As illustrated in Figure 1, various embodiments are directed to methods of predicting a medical outcome for an individual (e.g., patient). Many embodiments collect a patient sample at 102. Such samples can be collected at any time, such as before or after the event likely to trigger the medical outcome. At 104, many embodiments obtain omic data (e.g., proteomic, cytomic, and/or any other omic data) from the sample. Certain embodiments combine multiple omic data-e.g., plasma proteomics (e.g., analysis of plasma protein expression levels) and single-cell cytomics (e.g., single-cell analysis of circulating immune cell frequency and signaling activities)-as multi-omic data. Certain embodiments obtain clinical data for the individual. Clinical data in accordance with various embodiments includes one or more of medical history, age, weight, body mass index (BMI), sex/gender, current medications/supplements, functional status, emergency case, steroid use for chronic condition, ascites, disseminated cancer, diabetes, hypertension, congestive heart failure, dyspnea, smoking history, history of severe Chronic Obstructive Pulmonary Disease (COPD), dialysis, acute renal failure and/or any other relevant clinical data.

Additional embodiments generate a predictive model of the medical outcome, at 106. Many embodiments utilize a machine learning model, such as described herein. Various embodiments operate in a pipelined manner, such that as data, obtained or collected, are immediately sent to a machine learning model to generate a medical score. Some embodiments house the machine learning model locally, such that the medical score is generated without network communication, while some embodiments operate the machine learning model on a server or other remote device, such that clinical data and multi-omics data are transmitted via a network, and the medical score is returned to a medical professional/practitioner at their local institution, clinic, hospital, and/or other medical facility.

At 108, further embodiments adjust the treatment or care management of the individual based on the medical score. With this approach, therapeutic regimens and care management can be individualized and tailored according to predicted probability for a patient to develop a medical outcome, thereby providing a treatment or care management that is individually appropriate.

It should be noted that the embodiment illustrated in Figure 1 is illustrative of various steps, features, and details that can be implemented in various embodiments and is not intended to be exhaustive or limiting on all embodiments. Additionally, various embodiments may include additional steps, which are not described herein and/or fewer steps (e.g., omit certain steps) than illustrated and described. Various embodiments may also repeat certain steps, where additional data, prediction, or procedures can be updated for an individual, such as repeating generating a predictive model 106, to identify whether the medical score is more or less likely to develop in the individual. Further embodiments may also obtain samples or clinical data from a third party from a collaborating, subordinate, or other individual and/or obtaining a sample that has been stored or previously collected or obtained. Certain embodiments may even perform certain actions or features in a different order than illustrated or described and/or perform some actions or features simultaneously, relatively simultaneously (e.g., one action may begin or commence before another action has finished or completed).

The biological sample can be any suitable type that allows for the analysis of one or more cells, and proteins. Preferably the biological sample is a blood-based sample (such as whole blood, plasma, serum or a blood fraction), a tumor sample, or any other suitable biological sample such as a tissue biopsy. The biological sample can be obtained multiple times from an individual. Multiple samples can be obtained from different locations in the individual, at different times from the individual, or any combination thereof. In particular, the biological sample(s) for analysis of immune cell responses can be from any source that contains immune cells, such as blood or the PBMC fraction of blood. In particular, the biological sample(s) for proteomic analysis is the plasma or serum fraction of a blood sample.

According to certain embodiments, at least one biological sample is obtained prior to the event likely to trigger the medical outcome (e.g. prior to surgery, prior to drug administration, prior to onset of a clinical event in an individual at risk thereof). According to certain embodiments, at least one biological sample is obtained after the event likely to trigger the medical outcome (e.g. after surgery after drug administration, after onset of a clinical event in an individual). According to certain embodiments, at least one biological sample is obtained prior to the event likely to trigger the medical outcome and at least one biological sample is obtained after the event likely to trigger the medical outcome. Pre-event biological samples can be obtained 7 days, 6 days, 5 days, 4 days, 3 days, 2 days, 1 day, and/or 0 day (i.e., on the day of the event likely to trigger the medical outcome). Post-event biological samples can be obtained within 24 hours after the clinical event onset, including 0 hour, 1 hour, 3 hours, 6 hours, 8 hours, 10 hours, 12 hours, 16 hours, 18 hours, and/or 24 hours after.

In some embodiments, for generating cytomic features, samples are activated ex vivo, which as used herein refers to the contacting of a biological sample, e.g. a blood sample or cells derived therefrom, outside of the body with a stimulating agent. In some embodiments whole blood is preferred. The sample may be diluted or suspended in a suitable medium that maintains the viability of the cells, e.g. minimal media, PBS, etc. The sample can be fresh or frozen. Stimulating agents of interest include those agents that activate innate or adaptive cells, e.g. one or a combination of a TLR4 agonist such as LPS and/or IL-1β, IL-2, IL-4, IL-6, TNFα, IFNα, or GM-CSF, or PMA/ionomycin. Generally, the activation of cells ex vivo is compared to a negative control, e.g. medium only, or an agent that does not elicit activation. The cells are incubated for a period of time sufficient for activation of immune cells in the biological sample. For example, the time for activation can be up to about 1 hour, up to about 45 minutes, up to about 30 minutes, up to about 15 minutes, and may be up to about 10 minutes or up to about 5 minutes. In some embodiments the period of time is up to about 24 hours, or from about 5 to about 240 minutes. Following activation, the cells are fixed for analysis.

According to an embodiment, cytomic features are obtained by collecting whole blood from study subjects and stimulating individual aliquots, e.g. for 15 min at 37°C, with LPS (typically at about 1 ug/mL), IFN-α (typically at about 100 ng/mL), GM-CSF (typically at about 100 ng/mL), and a cocktail of IL-2, -4, and -6 (typically each at about 100 ng/mL) or left unstimulated. Samples are then fixed with proteomic stabilizer and stored at -80°C until further processing by antibody staining and mass cytometry analysis. Antibody staining uses a panel of antibodies for phenotyping of immune cell subsets and for the functional characterization of immune cell responses.

In many embodiments, cytomic, and proteomic features are detected using affinity reagents. "Affinity reagent", or "specific binding member" may be used to refer to an affinity reagent, such as an antibody, ligand, etc. that selectively binds to a protein or marker of the invention. The term "affinity reagent" includes any molecule, e.g., peptide, nucleic acid, small organic molecule. For some purposes, an affinity reagent selectively binds to a cell surface or intracellular marker, e.g. CD3, CD4, CD7, CD8, CD11b, CD11c, CD14, CD15, CD16, CD19, CD24, CD25, CD27, CD33, CD45, CD45RA, CD56, CD61, CD66, CD123, CD235ab, HLA-DR, CCR2, CCR7, TCRγδ, OLMF4, CRTH2, and CXCR4 and the like. For other purposes an affinity reagent selectively binds to a cellular signaling protein, particularly one which is capable of detecting an activation state of a signaling protein over another activation state of the signaling protein. Signaling proteins of interest include, without limitation, pSTAT3, pSTAT1, pCREB, pSTAT6, pPLCy2, pSTAT5, pSTAT4, pERK1/2, pP38, prpS6, pNF-κB (p65), pMAPKAPK2 (pMK2), pP90RSK, IκB, cPARP, FoxP3, and Tbet.

In some embodiments, proteomic features are measured and comprise measuring circulating extracellular proteins. Accordingly, other affinity reagents of interest bind to plasma proteins. Plasma protein targets of particular interest include IL-1β, ALK, WWOX, HSPH1, IRF6, CTNNA3, CCL3, sTREM1, ITM2A, TGFα, LIF, ADA, ITGB3, EIF5A, KRT19, and NTproBNP.

In some embodiments, cytomic features are measured and comprise measuring single cell levels of surface or intracellular proteins in an immune cell subset. Immune cell subsets include for instance neutrophils, granulocytes, basophils, monocytes, dendritic cells (DC) such as myeloid dendritic cells (mDC) or plasmacytoid dendritic cells (pDC), B-Cells or T-cells, such as regulatory T Cells (Tregs), naïve T Cells, memory T cells and NK-T cells. Immune cell subsets include more specifically neutrophils, granulocytes, basophils, CXCR4+neutrophils, OLMF4+neutrophils, CD14+CD16-classical monocytes (cMC), CD14-CD16+ nonclassical monocytes (ncMC), CD14+CD16+ intermediate monocytes (iMC), HLADR+CD11c+ myeloid dendritic cells (mDC), HLADR+CD123+ plasmacytoid dendritic cells (pDC), CD14+HLADR-CD11b+ monocytic myeloid derived suppressor cells (M-MDSC), CD3+CD56+ NK-T cells, CD7+CD19-CD3- NK cells, CD7+ CD56loCD16hi NK cells, CD7+CD56hiCD16lo NK cells, CD19+ B-Cells, CD19+CD38+ Plasma Cells, CD19+CD38- non-plasma B-Cells, CD4+ CD45RA+ naïve T Cells, CD4+ CD45RAmemory T cells, CD4+CD161+ Th17 cells, CD4+Tbet+ Th1 cells, CD4+CRTH2+ Th2 cells, CD3+TCRγδ+ γδT Cells, Th17 CD4+T cells, CD3+FoxP3+CD25+ regulatory T Cells (Tregs), CD8+ CD45RA+ naïve T Cells, and CD8+ CD45RA- memory T Cells.

In some embodiments both proteomic features and cytomic features are measured in a biological sample.

In some embodiments, metabolomic features are measured and comprise extracting metabolites from biological samples, such as plasma samples. Metabolite extraction can typically be performed using 1:1:1 acetone:acetonitrile:methanol, evaporated to dryness under nitrogen and reconstituted in 1:1 methanol:water before analysis. In some embodiments metabolic extracts are analyzed by combined chromatography/mass spectrometry analyses, e.g. using a broad-spectrum platform comprising two chromatographic systems (HILIC and RPLC) and two ionization modes (positive and negative).

In some embodiments, the affinity reagent is a peptide, polypeptide, oligopeptide or a protein, particularly antibodies, or an oligonucleotide, particularly aptamers and specific binding fragments and variants thereof. The peptide, polypeptide, oligopeptide or protein can be made up of naturally occurring amino acids and peptide bonds, or synthetic peptidomimetic structures. Thus "amino acid", or "peptide residue", as used herein include both naturally occurring and synthetic amino acids. Proteins including non-naturally occurring amino acids can be synthesized or in some cases, made recombinantly; see van Hest et al., FEBS Lett 428:(I-2) 68-70 May 22, 1998 and Tang et al., Abstr. Pap Am. Chem. S218: U138 Part 2 Aug. 22, 1999.

Many antibodies, many of which are commercially available (for example, see Cell Signaling Technology, www.cellsignal.com or Becton Dickinson, www.bd.com) have been produced which specifically bind to the phosphorylated isoform of a protein but do not specifically bind to a non-phosphorylated isoform of a protein. Many such antibodies have been produced for the study of signal transducing proteins which are reversibly phosphorylated. Particularly, many such antibodies have been produced which specifically bind to phosphorylated, activated isoforms of protein and plasma proteins. Examples of proteins that can be analyzed with the methods described herein include, but are not limited to, phospho (p) rpS6, pNF-κB (p65), pMAPKAPK2 (pMK2), pSTAT5, pSTAT1, pSTAT3, etc.

The methods the invention may utilize affinity reagents comprising a label, labeling element, or tag. By label or labeling element is meant a molecule that can be directly (i.e., a primary label) or indirectly (i.e., a secondary label) detected; for example, a label can be visualized and/or measured or otherwise identified so that its presence or absence can be known.

A compound can be directly or indirectly conjugated to a label which provides a detectable signal, e.g. non-radioactive isotopes, radioisotopes, fluorophores, enzymes, antibodies, oligonucleotides, particles such as magnetic particles, chemiluminescent molecules, molecules that can be detected by mass spec, or specific binding molecules, etc. Specific binding molecules include pairs, such as biotin and streptavidin, digoxin and anti-digoxin etc. Examples of labels include, but are not limited to, metal isotopes, optical fluorescent and chromogenic dyes including labels, label enzymes and radioisotopes. In some embodiments of the invention, these labels can be conjugated to the affinity reagents. In some embodiments, one or more affinity reagents are uniquely labeled.

Labels include optical labels such as fluorescent dyes or moieties. Fluorophores can be either "small molecule" fluors, or proteinaceous fluors (e.g. green fluorescent proteins and all variants thereof). In some embodiments, activation state-specific antibodies are labeled with quantum dots as disclosed by Chattopadhyay et al. (2006) Nat. Med. 12, 972-977. Quantum dot labeled antibodies can be used alone or they can be employed in conjunction with organic fluorochrome-conjugated antibodies to increase the total number of labels available. As the number of labeled antibodies increase so does the ability for subtyping known cell populations.

Antibodies can be labeled using chelated or caged lanthanides as disclosed by Erkki et al.(1988) J. Histochemistry Cytochemistry, 36:1449-1451, and U.S. Patent No. 7,018850. Other labels are tags suitable for Inductively Coupled Plasma Mass Spectrometer (ICP-MS) as disclosed in Tanner et al. (2007) Spectrochimica Acta Part B: Atomic Spectroscopy 62(3):188-195. Isotope labels suitable for mass cytometry may be used, for example as described in published application US 2012-0178183.

Alternatively, detection systems based on FRET can be used. FRET find use in the invention, for example, in detecting activation states that involve clustering or multimerization wherein the proximity of two FRET labels is altered due to activation. In some embodiments, at least two fluorescent labels are used which are members of a fluorescence resonance energy transfer (FRET) pair.

When using fluorescent labeled components in the methods and compositions of the present invention, it will be recognized that different types of fluorescent monitoring systems, e.g., cytometric measurement device systems, can be used to practice the invention. In some embodiments, flow cytometric systems are used or systems dedicated to high throughput screening, e.g. 96 well or greater microtiter plates. Methods of performing assays on fluorescent materials are well known in the art and are described in, e.g., Lakowicz, J. R., Principles of Fluorescence Spectroscopy, New York: Plenum Press (1983); Herman, B., Resonance energy transfer microscopy, in: Fluorescence Microscopy of Living Cells in Culture, Part B, Methods in Cell Biology, vol. 30, ed. Taylor, D. L. & Wang, Y.-L., San Diego:Academic Press (1989), pp. 219-243; Turro, N. J., Modern Molecular Photochemistry, Menlo Park: Benjamin/Cummings Publishing Col, Inc. (1978), pp. 296-361.

The detecting, sorting, or isolating step of the methods of the present invention can entail fluorescence-activated cell sorting (FACS) techniques, where FACS is used to select cells from the population containing a particular surface marker, or the selection step can entail the use of magnetically responsive particles as retrievable supports for target cell capture and/or background removal. A variety of FACS systems are known in the art and can be used in the methods of the invention (see e.g., WO99/54494, filed Apr. 16, 1999; U.S. Ser. No. 20010006787, filed Jul. 5, 2001).

In some embodiments, a FACS cell sorter (e.g. a FACSVantage™ Cell Sorter, Becton Dickinson Immunocytometry Systems, San Jose, Calif.) is used to sort and collect cells based on their activation profile (positive cells) in the presence or absence of an increase in activation level in an signaling protein in response to a modulator. Other flow cytometers that are commercially available include the LSR II and the Canto II both available from Becton Dickinson.

See Shapiro, Howard M., Practical Flow Cytometry, 4th Ed., John Wiley & Sons, Inc., 2003 for additional information on flow cytometers.

In some embodiments, the cells are first contacted with labeled activation state-specific affinity reagents (e.g. antibodies) directed against specific activation state of specific signaling proteins. In such an embodiment, the amount of bound affinity reagent on each cell can be measured by passing droplets containing the cells through the cell sorter. By imparting an electromagnetic charge to droplets containing the positive cells, the cells can be separated from other cells. The positively selected cells can then be harvested in sterile collection vessels. These cell-sorting procedures are described in detail, for example, in the FACSVantage™. Training Manual, with particular reference to sections 3-11 to 3-28 and 10-1 to 10-17.

In some embodiments, the activation level of an intracellular protein is measured using Inductively Coupled Plasma Mass Spectrometer (ICP-MS). An affinity reagent that has been labeled with a specific element binds to a marker of interest. When the cell is introduced into the ICP, it is atomized and ionized. The elemental composition of the cell, including the labeled affinity reagent that is bound to the signaling protein, is measured. The presence and intensity of the signals corresponding to the labels on the affinity reagent indicates the level of the signaling protein on that cell (Tanner et al. Spectrochimica Acta Part B: Atomic Spectroscopy, 2007 Mar;62(3):188-195.).

Mass cytometry, e.g. as described in the Examples provided herein, finds use on analysis. Mass cytometry, or CyTOF (DVS Sciences), is a variation of flow cytometry in which antibodies are labeled with heavy metal ion tags rather than fluorochromes. Readout is by time-of-flight mass spectrometry. This allows for the combination of many more antibody specificities in a single samples, without significant spillover between channels. For example, see Bodenmiller at a. (2012) Nature Biotechnology 30:858-867.

One or more cells or cell types or proteins can be isolated from body samples. The cells can be separated from body samples by red cell lysis, centrifugation, elutriation, density gradient separation, apheresis, affinity selection, panning, FACS, centrifugation with Hypaque, solid supports (magnetic beads, beads in columns, or other surfaces) with attached antibodies, etc. By using antibodies specific for markers identified with particular cell types, a relatively homogeneous population of cells can be obtained. Alternatively, a heterogeneous cell population can be used, e.g. circulating peripheral blood mononuclear cells.

In some embodiments, a phenotypic profile of a population of cells is determined by measuring the activation level of a signaling protein. The methods and compositions of the invention can be employed to examine and profile the status of any signaling protein in a cellular pathway, or collections of such signaling proteins. Single or multiple distinct pathways can be profiled (sequentially or simultaneously), or subsets of signaling proteins within a single pathway or across multiple pathways can be examined (sequentially or simultaneously).

In some embodiments, the basis for classifying cells is that the distribution of activation levels for one or more specific signaling proteins will differ among different phenotypes. A certain activation level, or more typically a range of activation levels for one or more signaling proteins seen in a cell or a population of cells, is indicative that that cell or population of cells belongs to a distinctive phenotype. Other measurements, such as cellular levels (e.g., expression levels) of biomolecules that may not contain signaling proteins, can also be used to classify cells in addition to activation levels of signaling proteins; it will be appreciated that these levels also will follow a distribution. Thus, the activation level or levels of one or more signaling proteins, optionally in conjunction with the level of one or more biomolecules that may or may not contain signaling proteins, of a cell or a population of cells can be used to classify a cell or a population of cells into a class. It is understood that activation levels can exist as a distribution and that an activation level of a particular element used to classify a cell can be a particular point on the distribution but more typically can be a portion of the distribution. In addition to activation levels of intracellular signaling proteins, levels of intracellular or extracellular biomolecules, e.g., proteins, can be used alone or in combination with activation states of signaling proteins to classify cells. Further, additional cellular elements, e.g., biomolecules or molecular complexes such as RNA, DNA, carbohydrates, metabolites, and the like, can be used in conjunction with activation states or expression levels in the classification of cells encompassed here.

In some embodiments of the invention, different gating strategies can be used in order to analyze a specific cell population (e.g., only CD4+ T cells) in a sample of mixed cell population. These gating strategies can be based on the presence of one or more specific surface markers. The following gate can differentiate between dead cells and live cells and the subsequent gating of live cells classifies them into, e.g. myeloid blasts, monocytes and lymphocytes. A clear comparison can be carried out by using two-dimensional contour plot representations, two-dimensional dot plot representations, and/or histograms.

The immune cells are analyzed for the presence of an activated form of a signaling protein of interest. Signaling proteins of interest include, without limitation, pMAPKAPK2 (pMK2), pP38, prpS6, pNF-κB (p65), IκB, pSTAT3, pSTAT1, pCREB, pSTAT6, pSTAT5, pERK. To determine if a change is significant the signal in a patient's baseline sample can be compared to a reference scale from a cohort of patients with known outcomes.

Samples may be obtained at one or more time points. Where a sample at a single time point is used, comparison is made to a reference "base line" level for the feature, which may be obtained from a normal control, a pre-determined level obtained from one or a population of individuals, from a negative control for ex vivo activation, and the like.

In some embodiments, the methods include the use of liquid handling components. The liquid handling systems can include robotic systems comprising any number of components. In addition, any or all of the steps outlined herein can be automated; thus, for example, the systems can be completely or partially automated. See USSN 61/048,657. As will be appreciated by those in the art, there are a wide variety of components which can be used, including, but not limited to, one or more robotic arms; plate handlers for the positioning of microplates; automated lid or cap handlers to remove and replace lids for wells on non-cross contamination plates; tip assemblies for sample distribution with disposable tips; washable tip assemblies for sample distribution; 96 well loading blocks; cooled reagent racks; microtiter plate pipette positions (optionally cooled); stacking towers for plates and tips; and computer systems.

Fully robotic or microfluidic systems include automated liquid-, particle-, cell- and organism-handling including high throughput pipetting to perform all steps of screening applications. This includes liquid, particle, cell, and organism manipulations such as aspiration, dispensing, mixing, diluting, washing, accurate volumetric transfers; retrieving, and discarding of pipet tips; and repetitive pipetting of identical volumes for multiple deliveries from a single sample aspiration. These manipulations are cross-contaminationfree liquid, particle, cell, and organism transfers. This instrument performs automated replication of microplate samples to filters, membranes, and/or daughter plates, highdensity transfers, full-plate serial dilutions, and high capacity operation.

In some embodiments, platforms for multi-well plates, multi-tubes, holders, cartridges, minitubes, deep-well plates, microfuge tubes, cryovials, square well plates, filters, chips, optic fibers, beads, and other solid-phase matrices or platform with various volumes are accommodated on an upgradable modular platform for additional capacity. This modular platform includes a variable speed orbital shaker, and multi-position work decks for source samples, sample and reagent dilution, assay plates, sample and reagent reservoirs, pipette tips, and an active wash station. In some embodiments, the methods of the invention include the use of a plate reader.

In some embodiments, interchangeable pipet heads (single or multi-channel) with single or multiple magnetic probes, affinity probes, or pipetters robotically manipulate the liquid, particles, cells, and organisms. Multi-well or multi-tube magnetic separators or platforms manipulate liquid, particles, cells, and organisms in single or multiple sample formats.

In some embodiments, the instrumentation will include a detector, which can be a wide variety of different detectors, depending on the labels and assay. In some embodiments, useful detectors include a microscope(s) with multiple channels of fluorescence; plate readers to provide fluorescent, ultraviolet and visible spectrophotometric detection with single and dual wavelength endpoint and kinetics capability, fluorescence resonance energy transfer (FRET), luminescence, quenching, two-photon excitation, and intensity redistribution; CCD cameras to capture and transform data and images into quantifiable formats; and a computer workstation.

In some embodiments, the robotic apparatus includes a central processing unit which communicates with a memory and a set of input/output devices (e.g., keyboard, mouse, monitor, printer, etc.) through a bus. Again, as outlined below, this can be in addition to or in place of the CPU for the multiplexing devices of the invention. The general interaction between a central processing unit, a memory, input/output devices, and a bus is known in the art. Thus, a variety of different procedures, depending on the experiments to be run, are stored in the CPU memory.

The differential presence of these markers is shown to provide for prognostic evaluations to detect individuals having a time to onset of labor. In general, such prognostic methods involve determining the presence or level of activated signaling proteins in an individual sample of immune cells. Detection can utilize one or a panel of specific binding members, e.g. a panel or cocktail of binding members specific for one, two, three, four, five or more markers.

Unless otherwise apparent from the context, all elements, steps or features described herein can be used in any combination with other elements, steps or features.

The present disclosure further refers the following patent and other publications: Alberts et al., The Molecular Biology of the Cell, 4th Ed., Garland Science, 2002; Vogelstein and Kinzler, The Genetic Basis of Human Cancer, 2d Ed., McGraw Hill, 2002; Michael, Biochemical Pathways, John Wiley and Sons, 1999; Weinberg, The Biology of Cancer, 2007; Immunobiology, Janeway et al. 7th Ed., Garland, and Leroith and Bondy, Growth Factors and Cytokines in Health and Disease, A Multi Volume Treatise, Volumes 1A and IB, Growth Factors, 1996.

General methods in molecular and cellular biochemistry can be found in such standard textbooks as Molecular Cloning: A Laboratory Manual, 3rd Ed. (Sambrook et al., Harbor Laboratory Press 2001); Short Protocols in Molecular Biology, 4th Ed. (Ausubel et al. eds., John Wiley & Sons 1999); Protein Methods (Bollag et al., John Wiley & Sons 1996); Nonviral Vectors for Gene Therapy (Wagner et al. eds., Academic Press 1999); Viral Vectors (Kaplift & Loewy eds., Academic Press 1995); Immunology Methods Manual (I. Lefkovits ed., Academic Press 1997); and Cell and Tissue Culture: Laboratory Procedures in Biotechnology (Doyle & Griffiths, John Wiley & Sons 1998). Reagents, cloning vectors, and kits for genetic manipulation referred to in this disclosure are available from commercial vendors such as BioRad, Stratagene, Invitrogen, Sigma-Aldrich, and ClonTech.

As an exemplary embodiment, the method for determining a medical outcome for an individual predicts the risk of developing of SSCs (including surgical site infection, wound dehiscence, abscess, or fistula formation). Once a classification or prognosis has been made, it can be provided to a patient or caregiver. The classification can provide prognostic information to guide the healthcare provider's or surgeon's clinical decision-making, such as delaying or adjusting the timing of surgery, adjusting the surgical approach, adjusting the type and timing of antibiotic and immune-modulatory regimens, personalizing or adjusting prehabilitation health optimization programs, planning for longer time in the hospital before or after surgery or planning for spending time in a managed care facility, and the like. Appropriate care can reduce the rate of SSCs, length of hospital stays, and/or the rate of readmission for patients following surgery.

For predicting SSC, samples can be collected at any time, such as before surgery or after surgery in the case of SSC. In some embodiments the sample is collected up to a week (7 days) before or after surgery. In certain embodiments, the sample is collected 1 day, 2 days, 3 days, 4 days, 5 days, 6 days, or 7 days before surgery, while some embodiments collect a sample 1 day, 2 days, 3 days, 4 days, 5 days, 6 days, or 7 days after surgery. Additional embodiments collect a sample day of surgery, including before and/or after surgery, including immediately before and/or after surgery. Certain embodiments collect multiple samples before, after, or before and after surgery, anesthesia, and/or any other procedural step included within a particular surgical or operational protocol.

Omic data (e.g., proteomic, cytomic, and/or any other omic data) are obtained from the sample(s). Certain embodiments combine multiple omic and cytomics as multi-omic data. Certain embodiments obtain clinical data for the individual. Clinical data in accordance with various embodiments includes one or more of medical history, age, weight, body mass index (BMI), sex/gender, current medications/supplements, functional status, emergency case, steroid use for chronic condition, ascites, disseminated cancer, diabetes, hypertension, congestive heart failure, dyspnea, smoking history, history of severe Chronic Obstructive Pulmonary Disease (COPD), dialysis, acute renal failure and/or any other relevant clinical data. Clinical data can also be derived from clinical risk scores such as the American Society of Anesthesiologist (ASA) or the American College of Surgeon (ACS) risk score.

**Machine learning methods for predicting medical outcomes**

To obtain a predictive model of a medical outcome, the method of the invention employs a machine learning method that integrates the single-cell analysis of immune cell responses using mass cytometry with the multiplex assessment of inflammatory plasma proteins in blood samples collected from patients. Many embodiments employ the Multi-Omic Bootstrap (MOB) machine learning method to predict the medical outcome. MOB, in accordance with various embodiments integrates one or more omic data categories (e.g., categories described herein) by extracting the most robust features from each data layer before combining these features and ensures stability of the features selected during statistical modeling of omic datasets.

The development of the stability selection method *(See e.g.,* Nicolai Meinshausen. Peter Bühlmann. Ann. Statist. 34 (3) 1436 - 1462, June 2006) is a key element in the development of the MOB algorithm. While the problem of variability is inherent and cannot be completely overcome, the stability selection can characterize this variation by considering the *frequency* at which each feature is chosen when multiple Lasso models are obtained on subsampled data. The selection frequency offers a quantitative measure into the importance of each feature that is readily interpretable from the biological standpoint. It has been shown that stability selection requires much weaker assumptions for asymptotically consistent variable selection compared to Lasso. Stated differently, stability selection, instead of selecting one model, subsamples data repeatedly and selects *stable variables,* that is, variables that occur in a large fraction of the resulting models. The chosen *stable variables* are defined by having selection frequency above a chosen threshold:

Equation (2)

$$\hat{S}_{stable} = \left\{ \beta_k : \max_{\lambda \in \Lambda} \Pi_k^\lambda > \pi_{th} \right\}$$

where $\Pi_k^\lambda$ is the selection frequency of feature k for the regularization parameter $\lambda$.

One of the difficulties of the previous prior art method is that it is difficult to assess noise. As the goal is to discriminate noisy variables from predictive ones, the use of negative control features is an appropriate approach to develop internal noise filter in the learning process. Negative control features designate synthetically made noisy features. One of the major contributions of this work is that, if built properly, it will be possible to adapt the thresholds previously mentioned from the distribution of the artificial features in the stability selection process. Two ways to generate these artificial features have been considered. Both techniques extend the initial input, ending up with an input matrix $(X, \tilde{X}) \in \mathbb{R}^{n \times 2p}$, where $\tilde{X}$ is the matrix of synthetic negative controls. The first technique called 'decoy' relies on a stochastic construction. Each synthetic feature is built by random permutation of its original counterpart (the permutation is independent for each synthetic feature). This process is done before each subsampling of the data. It is then possible to define a threshold from the behavior of the decoy feature in the stability selection, for instance:

<div align="center">Equation (3)</div>

$$\pi_{th} = c \times mean \max_{\lambda \in \Lambda} \Pi_{k+p}^{\lambda}$$

Where c is a ratio set by the user and *mean* $mean \max_{\lambda \in \Lambda} \Pi_{k+p}^{\lambda}$ is the mean of the maximum of selection frequency of the decoy features. The other technique uses model-X knockoffs *(See e.g.,* Candes, Emmanuel, et al. "Panning for gold:'model-X' knockoffs for high dimensional controlled variable selection." Journal of the Royal Statistical Society: Series B (Statistical Methodology) 80.3 (2018): 551-577) to build the synthetic negative controls. The construction allows to replicate the distribution of the original data (notably, the knockoffs correlation mimics the original one) and guarantees that the distribution of $\tilde{X}$ is orthogonal to the distribution of Y knowing X ($\tilde{X} \perp Y|X$). It is then possible to compare each pair of true/knockoffs variables after performing the stability selection and to select the feature k if:

<div align="center">Equation (4)</div>

$$\left( \max_{\lambda \in \Lambda} \Pi_{k}^{\lambda} - \max_{\lambda \in \Lambda} \Pi_{k+p}^{\lambda} \right) > cst$$

where $\Pi_{k}^{\lambda}$ and $\Pi_{k+p}^{\lambda}$ are the selection frequency of the feature k and its knockoff counterpart, and *cst* is a positive constant defined by the user.

The machine learning model is typically trained, using among other step a bootstrap procedure on a plurality of individual data layers. Each data layer represents one type of data from the plurality of possible features and at least one artificial feature. Each feature is for example chosen among a group consisting of: genomic, transcriptomic, proteomic, cytomic, metabolomic, clinical and demographic data.

Each data layer comprises data for a population of individuals, and each feature includes feature values for individuals in the population of individuals. During machine learning, for each data layer, the obtained feature values for the population of individuals are typically arranged in a matrix X with n rows and p columns, where each row corresponds to a respective individual and each column corresponds to a respective feature. In other words, the matrix X is a concatenation of p vectors, each one being related to a respective feature and containing n feature values, with typically one feature value for each individual.

For a respective data layer, each artificial feature, also called synthetic feature, is obtained from a non-artificial feature among the plurality of features, via a mathematical operation performed on the feature values of the non-artificial feature. The mathematical operation is for example chosen among the group consisting of: a permutation, a sampling with replacement, a sampling without replacement, a combination, a Knockoff sampling and an inference. The permutation is for instance a total permutation without replacement of the feature values. The sampling is typically a sampling with replacement of some of the feature values or a sampling without replacement of the feature values. The combination is for instance a linear combination of the feature values. The Knockoff sampling is for instance a Model-X knockoff applied to the feature values. The inference is typically a fit of a statistical distribution of the feature values, such as a Gaussian distribution, an exponential distribution, an uniform distribution or a Poisson distribution; and then inference sampling at random from it. The obtaining of artificial features is also called spike of artificial features, and corresponds to instruction 1 of Algorithm 2 in the below pseudo-code.

Advantageously, the obtaining of artificial features is done once during the bootstrap procedure. In other words, the artificial features are not obtained at each bootstrap sample generation, but only once for the whole bootstrap procedure, namely for or before the first bootstrap sample generation.

The skilled person will observe that when the artificial features are obtained at each bootstrap sample generation, it increases the complexity of the algorithm. According to the aforementioned advantage, to generate the artificial features are obtained, i.e. generated, for each omics only once before the stability selection process. Furthermore, generating the artificial features at each bootstrap iteration risks preventing some artificial features from being selected significantly enough. In some cases, it led to selecting artificial features at most 10% of the time, inducing a very low threshold and a very high number of selected features.

The model includes weights $\beta_i$ for a set of selected biological and clinical or demographic features, such weights $\beta_i$ being typically derived from initial weights wj repeatedly modified during the machine learning of the model.

During the machine learning and for each data layer, for every repetition of the bootstrap, the initial weights $w_j$ are computed for the plurality of features and the at least one artificial feature associated with that data layer, by using an initial statistical learning technique. The generation of the bootstrap samples, and respectively the estimation of the initial weights $w_j$, also called coefficients, correspond to instruction 4 of Algorithm 2 in the below pseudo-code.

The initial statistical learning technique is typically a sparse technique or a non-sparse technique. The initial statistical learning technique is for example chosen from among the group consisting in: a regression technique, a classification technique and an ensembling technique. Accordingly, the initial statistical learning technique is preferably chosen from among the group consisting of: a sparse regression technique, a sparse classification technique, a sparse ensembling technique, a non-sparse regression technique, a non-sparse classification technique and a non-sparse ensembling technique. The ensembling technique is for example a Random Forest technique or a XGBoost technique.

As an example, the initial statistical learning technique is therefore chosen from among the group consisting of: a linear or logistic linear regression technique with L1 or L2 regularization, such as the Lasso technique or the Elastic Net technique; (*see e.g.,* Tibshirani, Zou and Hastie cited above) a model adapting linear or logistic linear regression techniques with L1 or L2 regularization, such as the Bolasso technique (*see e.g.,* Bach, Francis R. "Bolasso: model consistent lasso estimation through the bootstrap." Proceedings of the 25th international conference on Machine learning. 2008), the relaxed Lasso (*see e.g.*, Meinshausen, Nicolai. "Relaxed lasso." Computational Statistics & Data Analysis 52.1 (2007): 374-393) the random-Lasso technique (*see e.g.,* Wang, Sijian, et al. "Random lasso." The annals of applied statistics 5.1 (2011): 468) the grouped-Lasso technique (*see e.g.*, Friedman, Jerome, Trevor Hastie, and Robert Tibshirani. Applications of the lasso and grouped lasso to the estimation of sparse graphical models. Technical report, Stanford University, 2010) the LARS technique (*see e.g.,* Eyraud, Remi, Colin De La Higuera, and Jean-Christophe Janodet. "LARS: A learning algorithm for rewriting systems." Machine Learning 66.1 (2007): 7-31) a linear or logistic linear regression technique without L1 or L2 regularization; a non-linear regression or classification technique with L1 or L2 regularization; a Decision Tree technique; a Random Forest technique; a Support Vector Machine technique, also called SVM technique; a Neural Network technique; and a Kernel Smoothing technique.

Then, at least one selected feature is determined, based on a statistical criteria depending on the computed initial weights $w_j$. The statistical criteria depends on significant weights among the computed initial weights $w_j$.

The significant weights are for example non-zero weights, when the initial statistical learning technique is a sparse regression technique, or weights above a predefined weight threshold, when the initial statistical learning technique is a non-sparse regression technique. The determination of the significant weights corresponds to instruction 6 of Algorithm 2 in the below pseudo-code.

As an example, the significant weights are non-zero weights, when the initial statistical learning technique is chosen from among the group consisting of: a linear or logistic linear regression technique with L1 or L2 regularization, such as the Lasso technique or the Elastic Net technique; a model adapting linear or logistic linear regression techniques with L1 or L2 regularization, such as the Bolasso technique, the relaxed Lasso, the random-Lasso technique, the grouped-Lasso technique, the LARS technique; a non-linear regression or classification technique with L1 or L2 regularization; and a Kernel Smoothing technique.

"Non-zero weight" refers to a weight which is in absolute value greater than a predefined very low threshold, such as $10^{-5}$, also noted 1e-5. Accordingly, "Non-zero weight" typically refers to a weight greater than $10^{-5}$ in absolute value.

Alternatively, the significant weights are weights above the predefined weight threshold, when the initial statistical learning technique is chosen from among the group consisting of: a linear or logistic linear regression technique without L1 or L2 regularization; a Decision Tree technique; a Random Forest technique; a Support Vector Machine technique; and a Neural Network technique. In the example of the Neural Network technique, the significant weights are weights above the predefined weight threshold on an initial layer of the corresponding neural network.

The skilled person will observe that the Support Vector Machine technique is considered as a sparse technique with support vectors, and the technique leads to only keeping the support vectors. The skilled person will also note that for the Decision Tree technique, the aforementioned weight corresponds to the feature importance, and accordingly that the significant weights are the features for which the split in the decision tree induces a certain decrease in impurity.

Optionally, the initial weights $w_j$ are further computed for a plurality of values of a hyperparameter A, the hyperparameter $\lambda$ being a parameter whose value is used to control the learning process. The hyperparameter $\lambda$ is typically a regularization coefficient used according to a respective mathematical norm in the context of a sparse initial technique. The mathematical norm is for example a P-norm, with P being an integer.

As an example, the hyperparameter $\lambda$ is an upper bound of the coefficient of the L1-norm of the initial weights $w_j$ when the initial statistical learning technique is the Lasso technique, where the L1-norm refers to the sum of all absolute values of the initial weights.

As another example, the hyperparameter $\lambda$ is an upper bound of the coefficient of the both the L1-norm sum of the initial weights $w_j$ and the L2-norm sum of the initial weights $w_j$ when the initial statistical learning technique is the Elastic Net technique, where the L1-norm is defined above and the L2-norm refers to the square root of the sum of all squared values of the initial weights.

As an advantageous variant to the above examples for determining the significant weights, the significant weights are weights above a computed weight threshold. According to this variant, the weight threshold computation includes calculating, for each hyperparameter value A, a ratio $FDR_\lambda$ depending on the number of artificial features with an associated weight above a unitary weight threshold $\beta_\lambda$ associated to hyperparameter value $\lambda$ divided by the number of features with an associated weight above said unitary weight threshold $\beta_\lambda$, each unitary weight threshold $\beta_\lambda$ being strictly positive and predefined. Each unitary weight threshold $\beta_\lambda$ is for example a non-zero weight or a weight above a predefined threshold. The computed weight threshold is then equal to the unitary weight threshold corresponding to the minimum calculated ratio among the calculated ratios $FDR_\lambda$ for the plurality $\Lambda$ of hyperparameter values $\lambda$.

According to this variant, each ratio $FDR_\lambda$ is for example calculated according to the following equation:

Equation (5)

$$FDR_\lambda = \frac{\#\{|\beta_i| > \beta_\lambda, \text{for i of artificial features}\} + 1}{\#\{|\beta_i| > \beta_\lambda, \text{for i of all features}\}}$$

where # represents the number of respective features verifying the condition $|\beta_i| > \beta_\lambda$; i is an index associated to each feature; $\beta_i$ represents the weight for the feature of index i; and $\beta_\lambda$ represents the unitary weight threshold associated to hyperparameter value $\lambda$.

For the feature selection, the statistical criteria depends for example on an occurrence frequency of the significant weights. As an example, the statistical criteria is that each feature is selected when its occurrence frequency is greater than a frequency threshold. In other words, a feature is selected when its occurrence frequency is greater than the frequency threshold.

According to the invention, the frequency threshold is computed according to the occurrence frequencies obtained for the artificial features, and so as to minimize a ratio depending on the number of selected artificial feature(s) divided by the number of selected features.

For each feature, to determine the occurrence frequency, a unitary occurrence frequency is calculated for each value of the hyperparameter A, the unitary occurrence frequency being equal to a number of the significant weights related to said feature for the successive bootstrap repetitions divided by the number bootstrap repetitions used for said feature. The occurrence frequency is then typically equal to the highest unitary occurrence frequency among the unitary occurrence frequencies calculated for all the values of the hyperparameter $\lambda$. The determination of each feature's occurrence frequency, also called selection frequency, corresponds to instructions 8 to 10 of Algorithm 2 in the below pseudo-code.

Advantageously, the frequency threshold computing includes calculating successively several ratios with respect to successive threshold values, each ratio $FDR_\tau$ depending on the number of selected artificial feature(s) with respect to a respective threshold value divided by the number of selected features with respect to said threshold value. The computed frequency threshold is then equal the threshold value corresponding to the minimum calculated ratio among the calculated ratios for the successive threshold values. The frequency threshold computing corresponds to instructions 11 to 20 of Algorithm 2 in the below pseudo-code.

Each ratio $FDR_\tau$ is for example calculated according to the following equation:

Equation (6)

$$FDR_\tau = \frac{FP_\tau + 1}{FP_\tau + TP_\tau}$$

where $FP_\tau$ is the number of selected artificial feature(s) and $TP_\tau$ is the number of selected non-artificial feature(s) for a given threshold value $\tau$, $FP_\tau + TP_\tau$ being therefore equal to the number of selected features.

Lastly, the feature selection is operated for each layer based on the statistical criteria. Therefore, the selected feature(s) are the one(s) which have their occurrence frequency greater than the frequency threshold. The feature selection corresponds to instructions 21 and 22 of Algorithm 2 in the below pseudo-code.

As an example, each value of the hyperparameter $\lambda$ is chosen according to a predefined scheme of values between the lower and upper bounds of the chosen value range for the hyperparameter $\lambda$. As a variant, the values of the hyperparameter $\lambda$ are evenly distributed between the lower and upper bounds of the chosen value range for the hyperparameter $\lambda$. The hyperparameter $\lambda$ is typically between 0.5 and 100 when the initial statistical learning technique is the Lasso technique or the Elastic Net technique.

For the bootstrapping process, the number bootstrap repetitions is typically between 50 and 100 000; preferably between 500 and 10 000; still preferably equal to 10 000.

During the machine learning, after the feature selection, the weights $\beta_i$ of the model are further computed using a final statistical learning technique on the data associated to the set of selected features.

The final statistical learning technique is typically a sparse technique or a non-sparse technique. The final statistical learning technique is for example chosen from among the group consisting in: a regression technique, a classification technique and an ensembling technique. Accordingly, the final statistical learning technique is preferably chosen from among the group consisting of: a sparse regression technique, a sparse classification technique, a sparse ensembling technique, a non-sparse regression technique, a non-sparse classification technique and a non-sparse ensembling technique. The ensembling technique is for example a Random Forest technique or a XGBoost technique.

As an example, the final statistical learning technique is therefore chosen from among the group consisting of: a linear or logistic linear regression technique with L1 or L2 regularization, such as the Lasso technique or the Elastic Net technique; a model adapting linear or logistic linear regression techniques with L1 or L2 regularization, such as the boLasso technique, the soft-Lasso technique, the random-Lasso technique, the grouped-Lasso technique, the LARS technique; a linear or logistic linear regression technique without L1 or L2 regularization; a non-linear regression or classification technique with L1 or L2 regularization; a Decision Tree technique; a Random Forest technique; a Support Vector Machine technique, also called SVM technique; a Neural Network technique; and a Kernel Smoothing technique.

As a variant, the final statistical learning technique is a non-linear technique, such as the Generalized Additive Model technique, also called GAM technique.

During a usage phase subsequent to the machine learning, the surgical risk score is computed according to the measured values of the individual for the set of selected features.

As an example, the surgical risk score is a probability calculated according to a weighted sum of the measured values multiplied by the respective weights $\beta_i$ for the set of selected features, when the final statistical learning technique is a respective classification technique.

According to this example, the surgical risk score is typically calculated with the following equation:

Equation (7)

$$P = \frac{Odd}{1 + Odd}$$

where P represents the surgical risk score, and
*Odd* is a term depending on the weighted sum.

As a further example, Odd is an exponential of the weighted sum. *Odd* is for instance calculated according to the following equation:

Equation (8)

$$Odd \;=\; exp(\beta_0 + \beta_1 X_1 + \cdots + \beta_{p_{stable}} X_{p_{stable}})$$

where *exp* represents the exponential function,
$\beta_0$ represents a predefined constant value,
$\beta_i$ represents the weight associated to a respective feature in the set of selected features,
$X_i$ represents the measured value of the individual associated to the respective feature, and
i is an index associated to each selected feature, i being an integer between 1 and $p_{stable}$, where $p_{stable}$ is the number of selected features for the respective layer.

The skilled person will notice that in the previous equation, the weights $\beta_i$ and the measured values $X_i$ may be negative values as well as positive values.

As another example, the surgical risk score is a term depending on a weighted sum of the measured values multiplied by the respective weights $\beta_i$ for the set of selected features, when the final statistical learning technique is a respective regression technique.

According to this other example, the surgical risk score is equal to an exponential of the weighted sum, typically calculated with the previous equation.

As a variant, when the final statistical learning technique is a non-linear technique, such as the GAM technique, the surgical risk score is typically calculated with the above equation (7).

According to this variant, the term *Odd* is for instance calculated according to the following equation:

Equation (9)

$$Odd = \beta_0 + f_1(X_1) + \cdots + f_{p_{stable}}(X_{p_{stable}})$$

where $\beta_0$ represents a predefined constant value,

$f_i$ represents a fitting function associated to a respective feature in the set of selected features,
$X_i$ represents the measured value of the individual associated to the respective feature, and
i is an index associated to each selected feature, i being an integer between 1 and $p_{stable}$, where $p_{stable}$ is the number of selected features for the respective layer.

The fitting functions $f_i$ are for example functions with a specified parametric form (e.g. a polynomial, or a non-penalized regression spline of a variable) or else are specified in a non-parametric, or semi-parametric way. To determine their form, the equivalent on the splines of the weights $\beta_i$ associated to the respective selected features are similarly as for a classical regression with the aforementioned equation (8).

As an optional addition, during the machine learning and before obtaining artificial features, additional values of the plurality of non-artificial features are generated based on the obtained values and using a data augmentation technique. According to this optional addition, the artificial features are then obtained according to both the obtained values and the generated additional values.

According to this optional addition, the data augmentation technique is typically a non-synthetic technique or a synthetic technique. The data augmentation technique is for example chosen among the group consisting of: SMOTE technique, ADASYN technique and SVMSMOTE technique.

According to this optional addition, for a given non-artificial feature, the less values have been obtained, the more additional values are generated.

According to this optional addition, this generation of additional values using the data augmentation technique is an optional additional step before the bootstrapping process. According to the above, this generation allows "augmenting" the initial input matrix X and the corresponding output vector Y with the data augmentation algorithm, namely increasing the respective sizes of the matrix X and the vector Y. If the matrix X is of size (n,p) and the vector Y is of size (n). This generation step leads to $X_{augmented}$ of size (n', p) and $Y_{augmented}$ of size (n') where n' > n.

This generation is preferably more sophisticated than the bootstrapping process. The goal is to 'augment' the inputs by creating synthetic samples, built using the obtained ones, and not by random duplication of samples. Indeed, if the non-artificial feature values would simply duplicated, the augmentation would not be fundamentally different from the bootstrapping process where non-artificial feature values may already be oversampled and/or duplicated. In the optional addition of data augmentation, the bootstrapping process will therefore be fed with new data points added to the original ones.

For classification, the data augmentation technique is for example the SMOTE technique, also called SMOTE algorithm or SMOTE. SMOTE first selects a minority class instance A at random and finds its K nearest minority class neighbors (using K Nearest Neighbor). The synthetic instance is then created by choosing one of the K nearest neighbors B at random and connecting A and B to form a line segment in the feature space. The synthetic instances are generated as a convex combination of the two chosen instances. The skilled person will notice that this technique is also a way of artificially balancing the classes. As a variant, the data augmentation technique is the ADASYN technique or the SVMSMOTE technique.

The MOB algorithm is typically applied to each layer independently.

As an example, in the case of the surgical site complications, namely when the medical outcome is SSC, the layers are for example the following ones: the immune cell frequency (containing 24 cell frequency features), the basal signaling activity of each cell subset (312 basal signaling features), the signaling response capacity to each stimulation condition (six data layers containing 312 features each), and the plasma proteomic (276 proteomic features).

As an example, for each layer, there are 41 samples. In other words, the number n of feature values for each feature is equal to 41 in this example. Accordingly, for the immune frequency layer, the dimensions of the matrix X are 41 samples (n) by 24 features (p). In the case of basal signaling, the matrix X is of dimension 41 x 312. Y is the vector of outcome values, namely the occurrence of SSC. This vector Y is in this case a vector of length 41.

Accordingly, one respective outcome value, i.e. one SSC value, is determined for each sample.

In this example, M is chosen equal to 10 000, which allows for enough sampling to derive an estimate of the frequency of selection over artificial features.

The chosen range value for the hyperparameter λ is between 0.5 and 100, with the statistical learning technique being the Lasso technique or the Elastic Net technique.

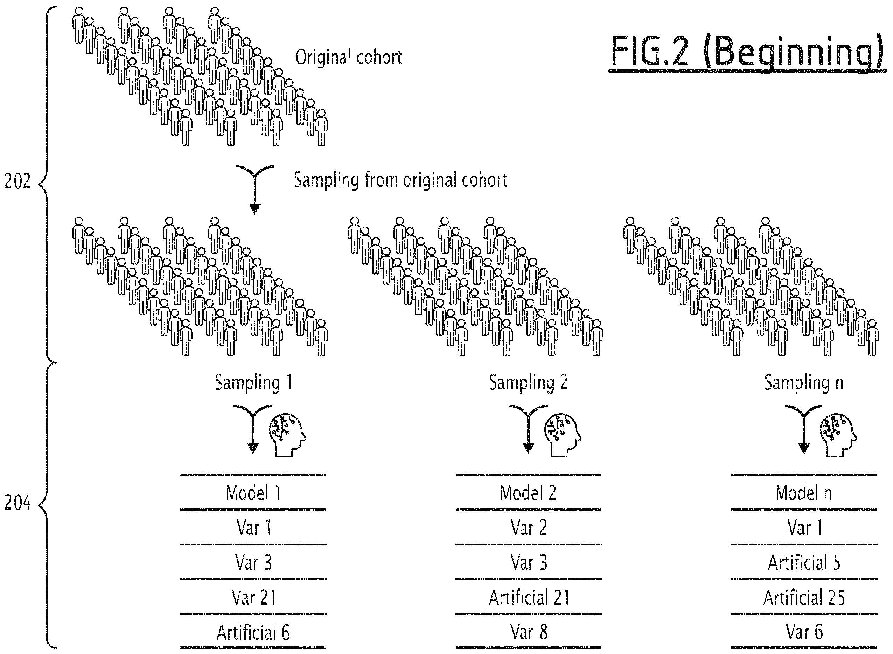

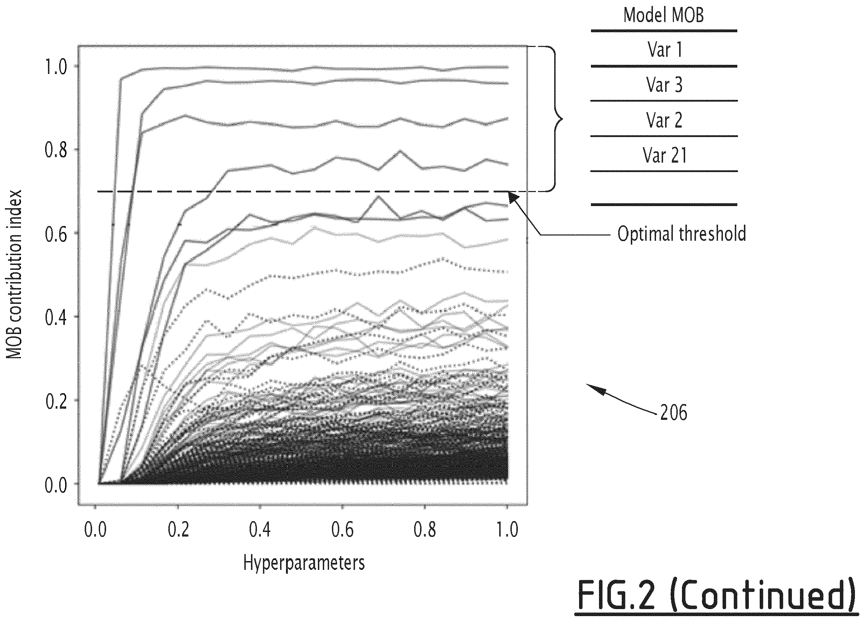

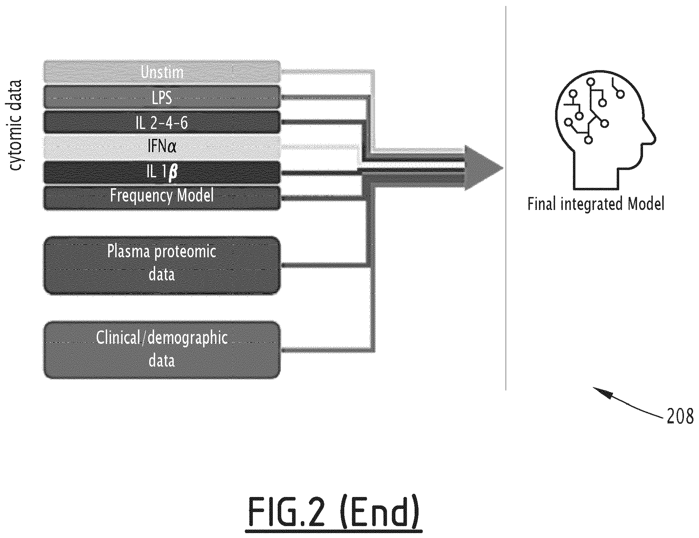

In the hereinafter example of Figure 2, the skilled person will notice that the mathematical operation used to obtain artificial features is the permutation or the sampling, and will understand that other mathematical operations would also be applicable, including the other ones mentioned in the above description, namely combination, Knockoff sampling and inference. Similarly, in these examples, the statistical learning techniques used to compute initial weights are sparse regression techniques, such as the Lasso and the Elastic Net, and the skilled person will also understand that other statistical learning techniques would also be applicable, including the other ones mentioned in the above description, namely non-sparse techniques and classification techniques. In this example, the significant weights are non-zero weights and the skilled person will also understand that other significant weights would also be applicable, such as weights above the predefined weight threshold, in accordance with the type of the initial statistical learning technique, as explained above. Alternatively, the significant weights are weights above the computed weight threshold.

Turning to Figure 2, the MOB algorithm is illustrated graphically. At 202, subsets are obtained from an original cohort with a procedure using repeated sampling with or without replacement on individual data layers. Artificial features are included by random sampling from the distribution of the original sample or by permutation and added to the original dataset. For each bootstrap, the artificial features are first built by selecting one by one the features (vectors of size p) of the original data matrix. To build an artificial feature, either a random permutation (equivalent to randomly drawing without replacement all the values of the vector) or a Knockoff sampling is typically performed. The process is repeated independently on each feature. The artificial features are then concatenated with the real features, and a draw with or without replacement samples is done from this concatenated dataset of features.

At 204, on each of the subsets, individual models are computed using, for example, a Lasso algorithm and features are selected based on contribution in the model (in the case of Lasso, non-zero features are selected), said contribution corresponding to the significant weights. At this stage of the process, a contributing feature has a non-zero coefficient when fitting the Lasso. This would be the same for any other technique inducing sparsity such as Elastic Net. For non-sparse regression techniques, an arbitrary contribution threshold would have to be defined. The threshold of selection can also be based on feature importance (such as in the case of Random Forest). The algorithm is adaptable to the machine learning technique used. Lasso is a well-known sparse regression technique, but other techniques are usable that select a subset of the original features. For instance, the Elastic Net (EN) as a combination of Lasso and Ridge would also work. Alternatively, a contributing feature has a coefficient, i.e. a weight, above the computed weight threshold.

At 206, using the features selected for each model and by hyperparameter, stability paths are obtained, such stability paths displaying the frequency of selection of each contributing feature (artificial or not). A stability path is, before any graphical transformation, the output matrix of the process. Its size is $(p, \#\Lambda)$. Each value $(feature_i, \lambda_j)$ corresponds to the frequency of selection of the $feature_i$ using the parameters $\lambda_j$. From this matrix, the path of each feature is displayed as shown in 206, where each line corresponds to the frequency of selection of each feature across all lambda tested. The distribution of selection of the artificial features are then used to estimate each ratio $FDR_{\tau}$, FDR meaning False Discovery Rate. A cutoff for relevant biological or clinical features is computed based on the estimated ratios FDR in the dataset. Typically, the frequency threshold is the one minimizing the FDR.

Only the relevant features from each layer are then used and combined in a final model for prediction of relevant medical outcomes. At 208, final integration of the model where each of the individual layers are combined with a process of selection similar to the process described in 202-206). In 208, all the top features are combined and used as predictors in a final layer. The final model uses the selected features obtained on each data layer using the MOB algorithm, in particular Algorithm 2 in the below pseudo-code. The input of the final model is therefore of size $(n, p_{stable})$, $p_{stable}$ being the number of selected features (all layers included). Note that $p_{stable}$ is significantly lower than the original feature space dimension. This reduced matrix is then trained for prediction of the outcome.

## Example of pseudo-code for the MOB algorithm

**Algorithm 1** STABL mutli-omics integration

**Input:** Let $X_{tot} = [X_{\Omega_1}, \ldots, X_{\Omega_N}]$ be the general input matrix, with $\Omega_i, \ldots, \Omega_N$ the different omics. Each $X_{\Omega_i}$ is a matrix of dimension $n$ (number of samples) by $p_i$ (number of features for this omic). $Y$ the vector of ouZtcome values (of size n), $M$ the number of bootstrap replicates, $\Lambda$ the vector of regularization parameters to test.

1: *StableFeatures* ← [ ]

2: **for** *i* in {1,...,*N*} **do**

3:         $StableFeatures_{\Omega_i} \leftarrow \boldsymbol{Algorithm2}(X_{\Omega_i}, Y, M, \Lambda)$

4:         Append   $StableFeatures_{\Omega_i}$ to *StableFeatures*

5: **end for**

6: Fit a machine learning algorithm on $X_{tot}[:,$ *StableFeatures*$]$ and $Y$ .


**Algorithm 2** STABL feature selection

**Input:**

$X_\Omega$ matrix of original features of the omic $\Omega$.

$X_\Omega$ is of dimension $n$ (number of samples) by $p$ (the number of original features).

$Y$ the vector of outcome values.

$M$ the number of bootstrap replicates.

$\Lambda$ the vector of regularization parameters to test.

1:         $X_\Omega^{concat} \leftarrow [X_\Omega, X_\Omega^{syn}]$. $X_\Omega^{syn}$ being the injected synthetic biomarkers by Knockoff sampling of $X_\Omega$ features. $X_\Omega^{concat}$ is now of dimension $n$ by $p + p'$ ($p'$ being the number of generated synthetic biomarkers).

2: **for** $\lambda$ in $\Lambda$ **do**

3:         **for** *i* in {1, ..., *M*} **do**

4: Generate bootstrap samples $(X_\Omega^i, Y^i)$ with size $\alpha n$ by $p + p'$. Where $\alpha$ is the bootstrap sampling proportion ($\alpha \in [0,1]$).

5: Fit a lasso model with the hyperparameters $\lambda$, or another sparse regression techniques model.

6: Extract the non-zero coefficients and their associated biomarkers (original of synthetic).

7:         **end for**

8: Build *freq*$_\lambda$ the vector of feature's selection frequencies at the current $\lambda$. *freq*$_\lambda$ is of size $(p + p')$

9: **end for**

10: $max_{freq} \leftarrow max\{freq_\lambda\}$ is the vector of size $p + p'$ containing the maximum, $\lambda \in \Lambda$ of selection frequency of each feature (original and synthetic) over all $\lambda$ values.

11: **for** $\tau \in [0,1]$ **do**

12:     $FP \leftarrow \#\{max_{freq}[syntheticfeatures] \geq \tau\}$.

13:     $TP \leftarrow \#\{max_{freq}[originalfeatures] \geq \tau\}$

14:     **if** $TP = 0$ and $FP = 0$ **then**

15:         $FDR \leftarrow 1$

16:     **else**

17:         $FDR \leftarrow \frac{FP+1}{FP+TP}$

18:     **end if**

19: **end for**

20: $\tau_{final} \leftarrow argmin_{\tau \in [0,1]}\{FDR\}$

21: $StableFeatures \leftarrow \{max_{freq}[originalfeatures] \geq \tau_{final}\}$

22: **return** $StableFeatures$

The above pseudo-code is an exemplary pseudo-code for MOB algorithm. The MOB uses a procedure of multiple resampling with or without replacement, called bootstrap, on individual data layers. In each data layer and for every repetition of the bootstrap, simulated features are spiked in the original dataset to estimate the robustness of selecting a biological feature compared to an artificial feature. An optimal cutoff for biological or clinical features is selected using the distribution of artificial features used to estimate the behavior of noise over biological or clinical features' robustness from the data layer. Then, the MOB algorithm selects the features above an optimal threshold calculated from the distribution of noise in each layer and builds a final model with the features from each data layer passing the optimal threshold of robustness. In many embodiments, performance is benchmarked, and stability is evaluated of feature selection on simulated data and biological data.

For the ease of reading, the MOB algorithm was splitted into two distinct parts, namely Algorithm 1, and then Algorithm 2. The first one, called Algorithm 1, concerns the overall workflow of MOB (omic separation and concatenation of stable features before the prediction part). The second algorithm, called Algorithm 2, details the bootstrap process that uses the synthetic features behavior and the FDR control to establish the frequency threshold for selection of original features, i.e. non-artificial features.

Algorithm 1

Pseudo-instruction 2: Looping over the N different omics (these omics are typically Proteomics, Metabolomics, Single Cell Proteomics and Clinical/Demographic Data). This corresponds to 108 in Figure 1.

Pseudo-instructions 3-4: The Algorithm 2 is applied on the previously concatenated matrix. The selected stable features are added to the general list of Stable Features.

Pseudo-instruction 6: At the end, the user can fit any machine learning algorithm (adapted to the task) on the input matrix made of the concatenation of stable features across all omics. The machine learning algorithm used in said instruction is typically the final statistical learning technique. In other words, each omic is considered, the most relevant features are extracted in each one of them and these features are then concatenated. The algorithm used at the end, such as the final statistical learning technique, is typically not a sparse regression technique as the most important part of the noise should already have been removed during the features extraction.

Algorithm 2

Pseudo-instruction 1: The permutation decoy or knockoff are obtained from the original dataset and the matrix $X_{\Omega i}^{concat}$ is a juxtaposition of the original matrix and the new matrix of artificial features. The number of artificial features $p_i'$ can vary but is typically chosen to match the number of original features included in the algorithm. For computational purposes, if $p_i$ is very large, a smaller number is chosen for $p_i'$ (particularly in the case of Knockoff where computing knockoffs for a value of $p_i > 1000$ can be quite intensive). The permutation decoy is simply the independent resampling of each feature and turns out to be particularly efficient on regression tasks as the chance of obtaining a feature with signal when resampling an original one randomly is quite rare. The knockoff sampling replicates the correlation structure of the data set and can be very useful for classification tasks where decoy by random permutation shows some limitations.

Pseudo-instruction 2: To properly probe selection behavior over the chosen algorithm hyperparameters, a grid search like scheme is operated to evaluate different combinations of hyperparameters, also allowing to plot a curve of "stability paths", as shown in 206 in Figure 2. It is also a way to avoid missing information if only a limited amount of hyperparameters is tested. The range of tested hyperparameters is preferably probed thoroughly to avoid artifacts (e.g., testing lambda = 0 for the lasso will select all features for all bootstrap procedures, leading to the case where the max of frequencies of selection are all equal to 1). When using the Lasso as the selection algorithm, it typically starts from the $\lambda_{max}$ $\left( = \frac{1}{n} ||X^t y||_\infty \right.$ in the case of regression, point where no feature is selected) value and ends with $\frac{\lambda_{max}}{100}$ or $\frac{\lambda_{max}}{10}$. This step represents the first for loop in the algorithm and yields results in the pseudo-instruction 10.

Pseudo-instruction 3: The iteration over the number of bootstrap repetitions is advantageous to get a proper evaluation of the sampling possibilities of artificial features selection. Index is tracked to see how sampling from the original distribution or via permutation behaves over multiple trials. For a regression task, the amount of bootstrap iterations required to have a stable behavior is around 100-300 iterations. For binary classification, this number can raise up to 1000 if the number of samples is quite small.

Pseudo-instructions 4-5: With a given number of spikes and for each chosen value of hyperparameters, the resampling procedure allows for an estimate of the model fit behavior and to select features that are the most robust to small changes in the dataset. By model fit behavior, it is referred to the assessment of the probability of selection by the Lasso for a given value of the hyperparameters. The bootstrap (resampling procedure) allows little perturbation in the original dataset and only the more robust features will be selected with a high frequency compared to others. The Elastic Net or Lasso algorithm tends to be very variable to small changes in the original cohort, especially in the sense that it can easily choses features that are not very robust, hence making biological interpretation and robustness over new cohorts difficult. In this setting, resampling creates small variations around the original cohort. This procedure can properly probe robustness in the feature selection.

Pseudo-instructions 6-8: The coefficients of each model are extracted, and with the sparsity induced by L1 regularization, a simple cutoff of non-zero coefficient is used (Typically 1e-5 in absolute value). This selection of top performing features at each iteration of the bootstrap procedure allows deriving a frequency of selection for each feature (original and synthetic) of the dataset.

Pseudo-instruction 10: *freq* is a matrix containing the selection frequencies of all features at each value of A. The "stability scores" of each feature (original or synthetic) are then built by taking the maximum of selection probability over all penalization coefficients.

Pseudo-instructions 11-20: The definition of the stability paths is used to estimate the FDR (False Discovery Rate) and then used to compute the cutoff for relevant features. The FDR is computed at several values of threshold value $\tau$ (typically from 0.3 to 1) as the ratio according to above equation (6). The cutoff, i.e. the frequency threshold, is then selected to be the one minimizing the FDR. As a further option, a desired level of FDR is set.

Pseudo-instructions 21-22: The selected features are the original features whose maximum of selection frequency is above the previously calculated optimal threshold, namely the frequency threshold.

As an additional aspect, the method according to the invention allows clustering. For each of the possible medical outcomes, the MOB algorithm is able to isolate the most predictive features for the observed target(s). From the subset of biomarkers identified, clusters are built for the observed samples. Those clusters are homogeneous groups regrouping samples with similar immunological profiles. In a multi-omic setting, different clustering are possible utilizing either single omic biomarkers or combinations of biomarkers from different omics. Once the most relevant features have been selected with the MOB algorithm, instead of integrating them in a supervised fashion with a learner, an alternative or an optional addition is to input the dataset with reduced dimensions into an unsupervised clustering algorithm, such as k-means, k-nearest neighbors, BIRCH, DBSCAN, Spectral Clustering or Gaussian Mixture Model. With some optimal cluster number metrics such as the silhouette score, it is then possible to define the optimal number of clusters and determine a profile based on the likelihood to be part of the defined clusters.

As another additional aspect, the method according to the invention allows obtaining interaction graph and dependence in multi-omic settings. Having identified the most predictive and important features to explain or monitor a medical outcome, such as a clinical outcome, it is possible to build an interaction graph to understand the dependencies between omics and their associated biomarkers. It is also possible to use the MOB algorithm to directly highlight the biomarkers explaining the variation of other omics (omic on omic analysis). From the selected features out of the algorithm, central nodes are defined in the correlation graph from which a threshold on the correlation coefficient is set. As an example, any of the features in the original dataset, i.e. the original features, that pass the threshold, is included in the graph and linked to the central node it is correlated to. Then from this graph, it is possible to propagate a variation that would occur on one of the central nodes by using these correlation coefficients.

## Computer Executed Embodiments

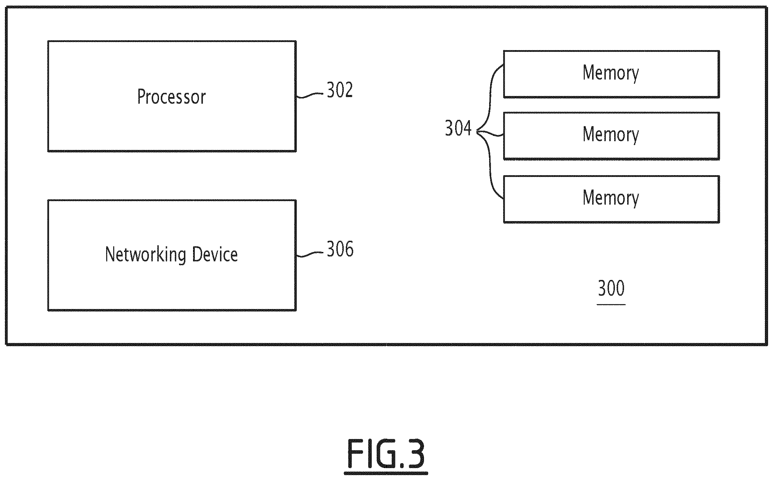

Processes that provide the methods and systems for generating a surgical risk score in accordance with some embodiments are executed by a computing device or computing system, such as a desktop computer, tablet, mobile device, laptop computer, notebook computer, server system, and/or any other device capable of performing one or more features, functions, methods, and/or steps as described herein. The relevant components in a computing device that can perform the processes in accordance with some embodiments are shown in Figure 3. One skilled in the art will recognize that computing devices or systems may include other components that are omitted for brevity without departing from described embodiments. A computing device 300 in accordance with such embodiments comprises a processor 302 and at least one memory 304. Memory 304 can be a non-volatile memory and/or a volatile memory, and the processor 302 is a processor, microprocessor, controller, or a combination of processors, microprocessor, and/or controllers that performs instructions stored in memory 304. Such instructions stored in the memory 304, when executed by the processor, can direct the processor, to perform one or more features, functions, methods, and/or steps as described herein. Any input information or data can be stored in the memory 304 -either the same memory or another memory. In accordance with various other embodiments, the computing device 300 may have hardware and/or firmware that can include the instructions and/or perform these processes.

Certain embodiments can include a networking device 306 to allow communication (wired, wireless, etc.) to another device, such as through a network, near-field communication, Bluetooth, infrared, radio frequency, and/or any other suitable communication system. Such systems can be beneficial for receiving data, information, or input (e.g., omic and/or clinical data) from another computing device and/or for transmitting data, information, or output (e.g., surgical risk score) to another device.

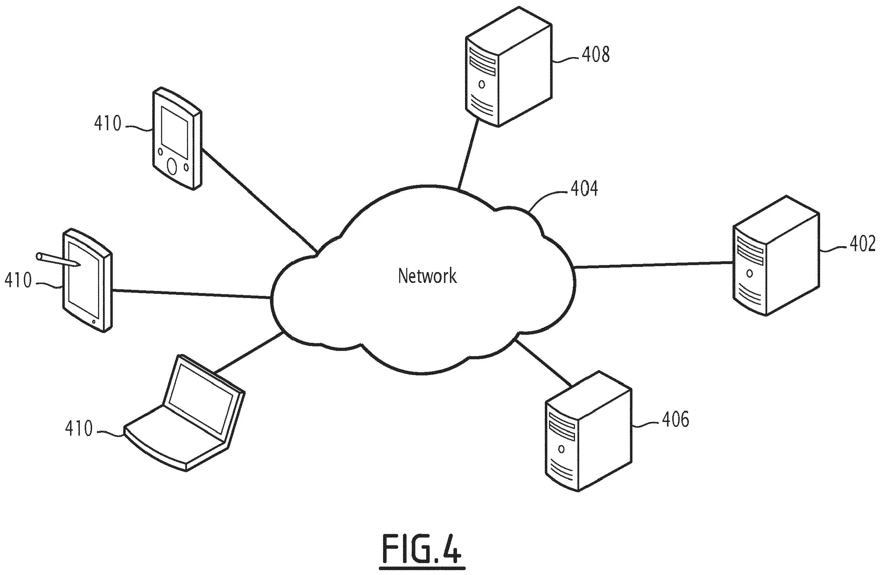

Turning to Figure 4, an embodiment with distributed computing devices is illustrated. Such embodiments may be useful where computing power is not possible at a local level, and a central computing device (e.g., server) performs one or more features, functions, methods, and/or steps described herein. In such embodiments, a computing device 402 (e.g., server) is connected to a network 404 (wired and/or wireless), where it can receive inputs from one or more computing devices, including clinical data from a records database or repository 406, omic data provided from a laboratory computing device 408, and/or any other relevant information from one or more other remote devices 410. Once computing device 402 performs one or more features, functions, methods, and/or steps described herein, any outputs can be transmitted to one or more computing devices 406, 408, 410 for entering into records, taking medical action-including (but not limited to) prehabilitation, delaying surgery, providing antibiotics-and/or any other action relevant to a surgical risk score. Such actions can be transmitted directly to a medical professional (e.g., via messaging, such as email, SMS, voice/vocal alert) for such action and/or entered into medical records.

In accordance with still other embodiments, the instructions for the processes can be stored in any of a variety of non-transitory computer readable media appropriate to a specific application.

## Examples

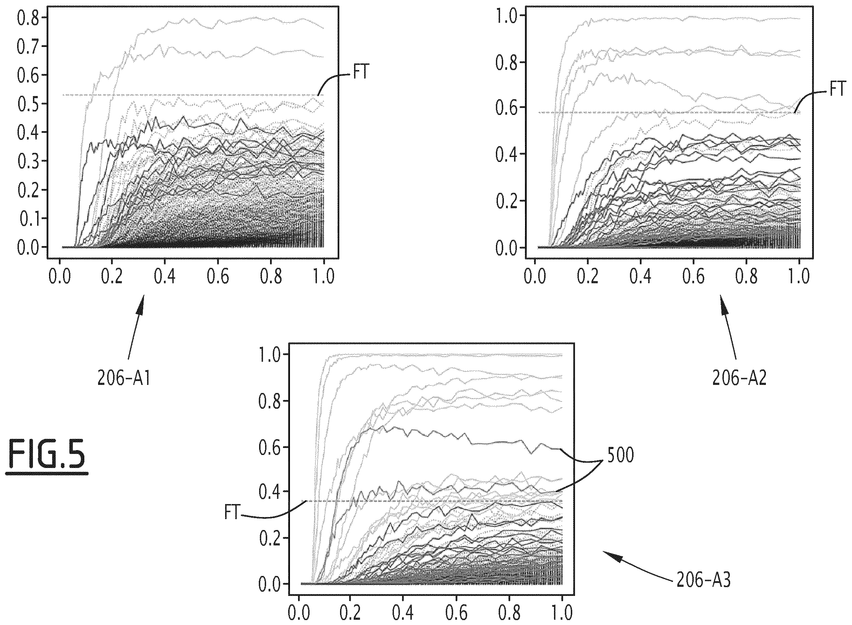

The Figures 5 to 7 show the results obtained with the method according to the invention for three different examples. In each example, the multi-omic input dataset consists of a large (>1000) number of biological features measured in the blood using a combination of omic technologies. Multi-omic dataset for the three examples are distinct but overlapping. The choice of data is primarily guided by technical factors (e.g. patient sample type availability and technology cost and availability) rather than by a preconceived understanding of the biology of related to the outcome of interest. As such, the input dataset is considered unbiased, as it is aimed to capture as many immune cell (CyTOF), or circulating proteomic or metabolomic features to allow the quasi-comprehensive assessment of patients inflammatory state.

Each one of the Figures 5 to 7 corresponds to a respective example, and on each one of Figures 5 to 7 are represented the stability paths for the features, each view denoted 206-Li being associated to a respective data layer, i.e. to a respective type features, L corresponding to a letter (A for the first example, B for the second example and C for the third example) and i corresponding to an integer index representing a respective data layer, with i between 1 and 3. The skilled person will observe that the views 206-Li in Figures 5 to 7 are similar to the view 206 in Figure 2. Each stability path is a line, or a curve, representing the frequency of selection of each feature across all hyperparameters $\lambda$ tested.

In the views 206-Li in each one of the Figures 5 to 7, the stability paths represented in continuous line correspond to original features, i.e. non-artificial features; the stability paths represented in dotted line correspond to artificial features; and the horizontal dotted line, denoted FT, represents the frequency threshold used for the features selection. In some cases, some stability paths are also denoted 500 and correspond to features selected only with the method according to the invention, and not with the prior art method, such as the Lasso method.

**First example** - **Prediction of onset of labor** - **Figure 5**

In the first example, illustrated in Figure 5, the determined medical outcome concerns the prediction of onset of labor, and more particularly the time between sample collection and "true" onset of spontaneous labor in term and preterm pregnancies.

For this first example, there are three types of input multi-omic data, i.e. three data layers, the first one corresponding cytometry (CyTOF) features, with 1502 single cell mass cytometry (CyTOF) features and the related stability paths shown in 206-A1; the second one corresponding proteomic features, with 1317 plasma proteomic features and the related stability paths shown in 206-A2; and the third one corresponding metabolomic features, with 3529 plasma metabolomic features and the related stability paths shown in 206-A3.

The method of the invention enabled defining a predictive model of labor onset based on a linear regression of selected features (Table 1)

Table 1: selected features and coefficients in the final Model

| Features (normalized : z-scored) | Linreg. coefficient |
|---|---|
| CD69negCD56loCD16negNK_STAT1_IFNa* | 3.8256328 |
| Granulocytes | -0.2530569 |
| Angiopoietin.2 | -3.1564398 |
| Cystatin.C | -1.1863203 |
| Siglec.6 | 3.86444983 |
| Activin.A | 3.31684556 |
| SPINT2 | 2.84972627 |
| Antithrombin.III | -2.2957575 |
| sTie.2 | -2.0792809 |
| uPA | 1.16898024 |
| IL.1.R4 | 3.8865809 |
| SLPI | 5.03109352 |
| MMP.12 | -2.8639794 |
| SLIK5 | 0.41190292 |
| VEGF121 | 0.54472036 |
| Angiogenin | 1.6518479 |
| GDF2 | -2.6356641 |
| HCG | -6.4302448 |
| PLXB2 | 2.43011954 |
| 4-Aminohippuric acid\|Pyridylacetylglycine\|Aminohippurate\|4-(Acetylamino)-3-Amino Benzoic Acid\|[[4-(Aminomethyl)Phenyl]Amino]Oxo-Acetic Acid | 1.11766052 |
| Deoxycorticosterone\|17-Hydroxyprogesterone\|11a-Hydroxyprogesterone\|11b-Hydroxyprogesterone\|[10]-Dehydroshogaol\|(4E,9a)-9-(3-Methyl-2E-pentenoyloxy)-4,10 (14)-oplopadien-3-one\|2-[2-(4-Hydroxy-3-methoxyphenyl)ethyl]-5-octylfuran\|Tussilagonone\|11-Hydroxy-delta-9-THC\|7-beta-Hydroxy-delta-9-THC\|8-Hydroxy-delta-9-THC\|8-beta-Hydroxy-delta-9-THC\|9-alpha,10-alpha-epoxyhexahydrocannabinol\|6(beta)-hydroxyprogesterone\|6-beta-hydroxyprogesterone\|16-O-Methylcafestol\|Jasmolin I\|Cannabielsoin | -0.7517418 |

(continued)

| Features (normalized : z-scored) | Linreg. coefficient |
|---|---|
| 3b, 15b, 17a-Trihydroxy-pregnenone\|3b,17a,21-Trihydroxypregnenone\|3a,21-Dihydroxy-5b-pregnane-11,20-dione\|11b,21-Dihydroxy-5b-pregnane-3,20-dione\|17a,21-Dihydroxypreg-nenolone\|7'-Carboxy-alpha-chromanol\|[10]-Gingerdione\|[8]-Paradyl acetate\|9alpha-(3- | 3.23826847 |
| Methyl-2E-pentenoyloxy)-4S-hydroxy-10(14)-oplopen-3-one\|10-Gingerdione | |
| 4-(1R,3AS,4R,8AS,8BR)-[1-Difluoromethyl-2-(4-Fluorobenzyl)-3-Oxodecahydropyrrolo[3,4-A]Pyrrolizin-4-YL]Benzamidine\|Austalide J\|1-{3-oxo-3-[(2S)-2-(pyrrolidin-1-ylcarbonyl)pyrrolidin-1-yl]propyl}-3-phenylquinoxalin-2(1H)-one\|[1-(4-Fluorobenzyl)Cyclobutyl]Methyl (1s)-1-[Oxo(1h-Pyrazol-5-Ylamino)Acetyl\|Pentylcarbamate | 7.92509697 |
| Neryl rhamnosyl-glucoside\|xi-Linalool 3-[rhamnosyl-(1->6)-glucoside]\|Geranyl rhamnosyl-glucoside | -0.2681976 |
| *CD69-CD56$^{low}$CD16$^-$ NK cells stimulated with IFNa and expressing STAT1 | |

As an example, several metrics are used to compare the performance of the method according to the invention with the prior art method.

Median of the Jaccard index (JI in hereinafter Tables) for stability measurement

When repeating an experiment 50 times, the Jaccard matrix, denoted Jaccard$_{Stability}$, is computed. This matrix is of size (n experiments, n experiments) (50 by 50 in this case).

$jaccard_{Stability}[i,j] = Jaccard_{index}(\hat{S}_i, \hat{S}_j)$ with $Jaccard_{index}(A,B) = \left|\frac{A \cap B}{A \cup B}\right|$ and $\hat{S}_i$ being the set of selected features at experiment n°i. Conventionally, if |A| = |B| = 0 then Jaccard$_{index}$(A,B) = 0. Finally, the median and the interquartile range of the upper triangle of the Jaccard matrix Jaccard$_{Stability}$ are used to assess the stability performance i.e. the ability to select the same set of features from one experiment to the other.

Median of the Jaccard index for feature selection performance

At each experiment, $Jaccard_{index}(\hat{S}_i, S)$ is computed, S being the set of truly predictive features or ground truth, known in synthetically generated data sets. The performance of feature selection is assessed by taking the median and the interquartile range across all experiments.

R$^2$-Score, RMSE and Mean Absolute Error (MAE)

As the goal is to prove the ability of choosing a better set of predictive features without losing regression power, the well-known metrics R$^2$-Score, RMSE and Mean Absolute Error are also monitored to check this. To compute those scores with the model according to the invention, also called STABL, a final model layer is added after the feature selection to predict the outcome using the features selected by the model STABL. Those models are generally linear models or random forests.

R$^2$-Score, Root Mean Square Error (RMSE) and Mean Absolute Error (MAE) typically verify the following equations:

$$R^2 = 1 - \frac{\sum(y_i - y_{pred_i})^2}{\sum(y_i - \bar{y})^2}$$

$$RMSE = \sum(y_i - y_{pred_i})^2$$

$$MAE = \sum |y_i - y_{pred_i}|$$

with y and $y_{pred}$ respectively, the true labels and predicted values; and
$\bar{y}$ representing the mean of the terms $y_i$.

For the first example, and for each data layer, the compared performances of the method according to the invention and of the prior art method are summarized in the hereinafter Table 2.

Table 2: STABL (model according to the invention) vs Lasso (prior art model) on onset of labor (OOL)

| Model | Average number of selected features | JI | $R^2$ | RMSE | MAE |
|---|---|---|---|---|---|
| CyTOF | | | | | |
| Lasso (prior art) | 20.3 | 0.24 [0.18, 0.3] | 0.37 [0.23, 0.46] | 26.66±6.7 | 21.0±3.2 |
| STABL (invention) | 3.6 | 0.33 [0.19, 0.39] | 0.32 [0.16, 0.42] | 27.6±2.9 | 21.8±2.3 |
| Proteins | | | | | |
| Lasso (prior art) | 29.0 | 0.28 [0.19, 0.39] | 0.63±0.22 | 18.5±4.6 | 13.9±2.7 |
| STABL (invention) | 12.1 | 0.5 [0.42, 0.58] | 0.73±0.11 | 16.0±3.1 | 12.2±2.0 |
| Metabolomics | | | | | |
| Lasso (prior art) | 42.6 | 0.20 [0.14, 0.27] | 0.58±0.26 | 19.6±5.3 | 14.1±2.53 |
| STABL (invention) | 8.5 | 0.43 [0.33, 0.55] | 0.70±0.12 | 16.9±3.1 | 12.6±2.2 |

The skilled person will observe that $R^2$-scores are given in median and interquartile range for the CyTOF as high negative values are distorting the mean of the distribution.

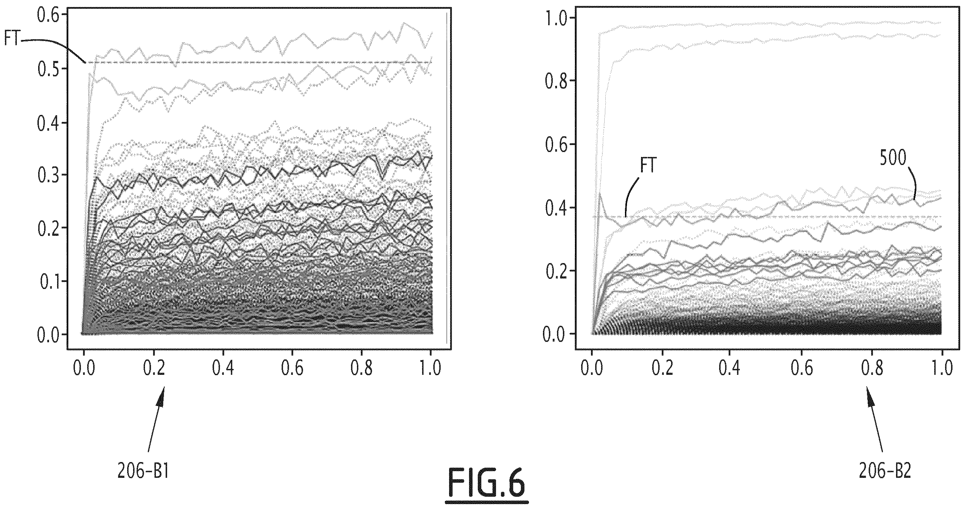

**Second example** - **Diagnosis of COVID19 severity** - **Figure 6**

In the second example, illustrated in Figure 6, the determined medical outcome concerns the diagnosis of COVID19 severity, and more particularly a classification of this severity into three categories: mild, moderate or severe COVID19.

For this second example, there are two types of input multi-omic data, i.e. two data layers, the first one corresponding cytometry (CyTOF) features, with 2664 single cell mass cytometry (CyTOF) features and the related stability paths shown in 206-B1; the second one corresponding proteomic features, with 1464 plasma proteomic features and the related stability paths shown in 206-B2.

The method of the invention enabled defining a predictive model of COVID19 severity (Table 3).

Table 3: selected features and coefficients in the final Model

| Features (normalized : z-scored) | Logit coefficient |
|---|---|
| IgMnBcell_IgM_Unstim | -15.989829737993200 |
| CD8Tmem_S6_Unstim | -24.53348653793930 |
| CBLN4 | 35.61200366111890 |
| CPVL | 16.850576798401400 |
| FCN2 | 10.575051476416300 |

(continued)

| Features (normalized : z-scored) | Logit coefficient |
|---|---|
| GALNT10 | -4.796912554387870 |
| IDS | 35.99668039480750 |
| IgMnBcell_IgM_Unstim: unstimulated IgM negative B cell<br>CD8Tmem_S6_Unstim: unstimulated CD8 memory T cell expressing S6 | |

For the second example, and for each data layer, the compared performances of the method according to the invention and of the prior art method are summarized in the hereinafter Table 4.

Table 4: STABL (model according to the invention) vs Lasso (prior art model) on COVID-19 data set

| Model | Average number of selected features | JI | AUC |
|---|---|---|---|
| | CyTOF | | |
| Lasso (prior art) | 22.9 | 0.20 [0.15, 0.26] | 0.71±0.13 |
| STABL (invention) | 7.0 | 0.25 [0.18, 0.35] | 0.72±0.13 |
| | Proteins | | |
| Lasso (prior art) | 21.6 | 0.21 [0.17, 0.27] | 0.95±0.07 |
| STABL (invention) | 7.0 | 0.33 [0.25, 0.42] | 0.95±0.07 |

The same results for COVID-19 and Preeclampsia prediction with CFRNA can be seen in Table 2 and Table 4 respectively.

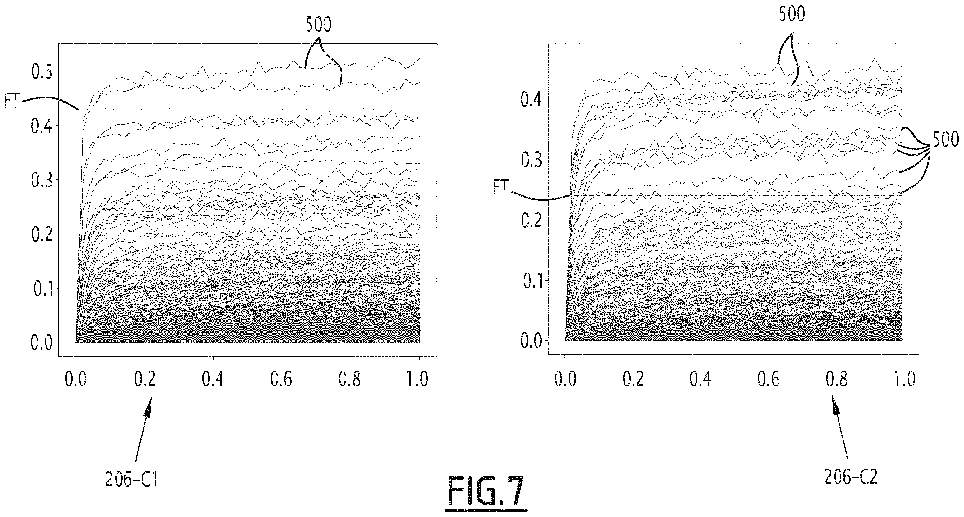

**Third example** - **Prediction of surgical site infection** - **Figure 7**

In the third example, illustrated in Figure 7, the determined medical outcome concerns the prediction of surgical site infection, and more particularly an incidence of surgical site infection after major abdominal surgery.
For this third example, there are two types of input multi-omic data, i.e. two data layers, the first one corresponding cytometry (CyTOF) features, with 1125 single cell mass cytometry (CyTOF) features and the related stability paths shown in 206-C1; the second one corresponding proteomic features, with 721 plasma proteomic features and the related stability paths shown in 206-C2.
The method of the invention enabled defining a predictive model of SSC (Table 5).

Table 5: selected features and coefficients in the final Model

| Features (normalized : z-scored) | Logit coefficient |
|---|---|
| unstim_CD4Trm_Frequency | 38.554347558933300 |
| unstim_Tregnaive_Frequency | 74.88393222549830 |
| ALK | 90.85902466649080 |
| ARL 11.00 | 112.14241875562700 |
| ASAH1 | 20.25304200631730 |
| CCL3 | 104.05261241712700 |
| CTNNA3 | -28.992255971841100 |
| HSPH1 | -5.021457027417500 |
| IL1B | 67.1024442417372 |
| LDLR | 37.50476896504980 |
| WWOX | 44.20239157536080 |

For the third example, and for each data layer, the compared performances of the method according to the invention and of the prior art method are summarized in the hereinafter Table 6.

Table 6: STABL (model according to the invention) vs Lasso (prior art model) on BioBank data set

| Model | Average number of selected features | JI | AUC |
|---|---|---|---|
| | CyTOF | | |
| Lasso (prior art) | 24.3 | 0.07 [0.01, 0.2] | 0.42±0.2 |
| STABL (invention) | 2.1 | 0.5 [0.33, 1.0] | 0.62±0.18 |
| | Proteins | | |
| Lasso (prior art) | 38.5 | 0.25 [0.18, 0.36] | 0.70±0.14 |
| STABL (invention) | 6.4 | 0.21 [0.15, 0.26] | 0.70±0.14 |

**Claims**

1. A method for determining a medical outcome for an individual, comprising:

- obtaining or having obtained values of a plurality of features, wherein the plurality of features includes omic biological features and clinical features;
- computing a medical score related to the medical outcome for the individual based on the plurality of features using a model obtained via a machine learning technique; and
- providing an assessment of the individual's medical outcome based on the computed medical score;
wherein the machine learning model is trained using a bootstrap procedure on a plurality of individual data layers, wherein each data layer represents one type of data from the plurality of features and at least one artificial feature;
wherein the model includes weights ($\beta_i$) for a set of selected biological and clinical or demographic features;
during the machine learning and for each data layer, for every repetition of the bootstrap, initial weights ($w_j$) are computed for the plurality of features and/or the at least one artificial feature associated with that data layer using an initial statistical learning technique, and at least one feature is selected;
wherein a feature is selected when an occurrence frequency associated to said feature of significant weights among the computed initial weights ($w_j$) is greater than a frequency threshold, the frequency threshold being computed according to the occurrence frequencies obtained for the artificial features; and
wherein the frequency threshold is computed to minimize a ratio depending on the number of selected artificial feature(s) divided by the number of selected features.

2. The method according to claim 1, wherein the initial weights ($w_j$) are further computed for a plurality of values of a hyperparameter, wherein the hyperparameter is a parameter whose value is used to control the learning process;

the hyperparameter being preferably a regularization coefficient used according to a respective mathematical norm in the context of a sparse initial technique;
the mathematical norm being further preferably a p-norm, with p being an integer.

3. The method according to claim 2, wherein for each feature, a unitary occurrence frequency is calculated for each hyperparameter value ($\lambda$) and is equal to a number of the significant weights related to said feature for the successive bootstrap repetitions divided by the number bootstrap repetitions;
the occurrence frequency being preferably equal to the highest unitary occurrence frequency among the unitary occurrence frequencies calculated for the plurality ($\Lambda$) of hyperparameter values ($\lambda$).

4. The method according to any one of claims 1 to 3, wherein the frequency threshold computing includes:

+ calculating successively several ratios with respect to successive threshold values, each ratio ($FDR_\tau$) depending on the number of selected artificial feature(s) with respect to a respective threshold value divided by the number of selected features with respect to said threshold value,
+ the computed frequency threshold being equal the threshold value corresponding to the minimum calculated ratio among the calculated ratios for the successive threshold values;
each ratio ($FDR_\tau$) being preferably calculated according to the following equation:

$$FDR_\tau = \frac{FP_\tau + 1}{FP_\tau + TP_\tau}$$

where $FP_\tau$ is the number of selected artificial feature(s) and $TP_\tau$ is the number of selected non-artificial feature(s) for a given threshold value $\tau$, $FP_\tau + TP_\tau$ being therefore equal to the number of selected features.

5. The method according to any one of the preceding claims, wherein the initial statistical learning technique is selected from the group consisting in: a regression technique, a classification technique and an ensembling technique;

   the initial statistical learning technique being preferably selected from a sparse technique and a non-sparse technique;
   the sparse technique being further preferably selected from a Lasso technique and an Elastic Net technique;
   the ensembling technique being further preferably selected from a Random Forest technique or a XGBoost technique.

6. The method according to claim 5, wherein the significant weights are non-zero weights, when the initial statistical learning technique is a sparse regression technique; and
   wherein the significant weights are weights above a predefined weight threshold, when the initial statistical learning technique is a non-sparse regression technique.

7. The method according to any one of claims 1 to 5, taken with claims 2 and 5, wherein the significant weights are weights above a computed weight threshold, and the weight threshold computation includes:

   + calculating, for each hyperparameter value ($\lambda$), a ratio ($FDR_\lambda$) depending on the number of artificial feature(s) with an associated weight above a unitary weight threshold ($\beta_\lambda$) associated to hyperparameter value ($\lambda$) divided by the number of features with an associated weight above said unitary weight threshold ($\beta_\lambda$), each unitary weight threshold ($\beta_\lambda$) being strictly positive and predefined,
   + the computed weight threshold being equal to the unitary weight threshold corresponding to the minimum calculated ratio among the calculated ratios ($FDR_\lambda$) for the plurality ($\Lambda$) of hyperparameter values ($\lambda$);
   each ratio ($FDR_\lambda$) being preferably calculated according to the following equation:

$$FDR_\lambda = \frac{\#\{|\beta_i| > \beta_\lambda, \text{for i of artificial features}\} + 1}{\#\{|\beta_i| > \beta_\lambda, \text{for i of all features}\}}$$

   where # represents the number of respective features verifying the condition $|\beta_i| > \beta_\lambda$; i is an index associated to each feature; $\beta_i$ represents the weight for the feature of index i; and $\beta_\lambda$ represents the unitary weight threshold associated to hyperparameter value $\lambda$.

8. The method according to any one of the preceding claims, taken with claims 2 and 5, wherein the hyperparameter is an upper bound of the coefficient of the L1-norm of the initial weights ($w_j$) when the initial statistical learning technique is the Lasso technique, wherein the L1-norm refers to the sum of all absolute values of the initial weights; and wherein the hyperparameter is an upper bound of the coefficient of the to both the L1 - norm sum of the initial weights ($w_j$) and the L2-norm sum of the initial weights ($w_j$) when the initial statistical learning technique is the Elastic Net technique, wherein the L1-norm refers to the sum of all absolute values of the initial weights, and L2-norm refers to the square root of the sum of all squared values of the initial weights.

9. The method according to any one of the preceding claims, wherein omic biological features comprise at least one feature selected from the group consisting of a genomic feature, a transcriptomic feature, a proteomic feature, a cytomic feature, and a metabolomic feature;

   the plurality of features preferably further including clinical and/or demographic features;
   each type being further preferably selected from the group consisting of genomic, transcriptomic, proteomic, cytomic, metabolomic, clinical and demographic data.

10. The method according to any one of the preceding claims, wherein each data layer comprises data for a population of individuals; each feature includes feature values for individuals in the population of individuals; and

for a respective data layer, each artificial feature is obtained from a non-artificial feature among the plurality of features, via a mathematical operation performed on the feature values of the non-artificial feature;
the mathematical operation being preferably chosen among the group consisting of: a permutation, a sampling with replacement, a sampling without replacement, a combination, a Knockoff sampling and an inference;
obtaining artificial features being preferably done once during the bootstrap procedure.

11. The method according to claim 10, wherein during the machine learning, the method further comprises, before obtaining artificial features:

- generating additional values of the plurality of non-artificial features based on the obtained values and using a data augmentation technique;
the artificial features being then obtained according to both the obtained values and the generated additional values;
the data augmentation technique being preferably chosen among a non-synthetic technique and a synthetic technique;
the data augmentation technique being further preferably chosen among the group consisting of: SMOTE technique, ADASYN technique and SVMSMOTE technique;
for a given non-artificial feature, the less values have been obtained, the more additional values being preferably generated.

12. The method according to any one of the preceding claims, wherein during the machine learning, the weights ($\beta_i$) of the model are further computed using a final statistical learning technique on the data associated to the set of selected features;

the final statistical learning technique being preferably selected from the group consisting in: a regression technique, a classification technique and an ensembling technique;
the final statistical learning technique being preferably selected from a sparse technique and a non-sparse technique;
the sparse technique being further preferably selected from a Lasso technique and an Elastic Net technique, or else from a non-linear technique, such as the Generalized Additive Model (GAM) technique;
the ensembling technique being further preferably selected from a Random Forest technique or a XGBoost technique.

13. The method according to any one of the preceding claims, wherein during a usage phase subsequent to the machine learning, the medical score is computed according to the measured values of the individual for the set of selected features;

the medical score being preferably a probability calculated according to a weighted sum of the measured values multiplied by the respective weights ($\beta_i$) for the set of selected features, when the final statistical learning technique is the classification technique; the medical score being further preferably calculated according to the following equation:

$$P = \frac{Odd}{1 + Odd}$$

where P represents the medical score, and
*Odd* is a term depending on the weighted sum; *Odd* being for example an exponential of the weighted sum;

the medical score being preferably a term depending on a weighted sum of the measured values multiplied by the respective weights ($\beta i$) for the set of selected features, when the final statistical learning technique is the regression technique; the medical score being further preferably equal to an exponential of the weighted sum.

14. The method of any one of the preceding claims, wherein the medical outcome is selected from the group consisting of postoperative complications, clinical event, drug efficiency or patient's responsiveness to treatment, drug side effects and allergies, surgical recovery, auto-immune and chronic diseases diagnosis, diet efficiency, impact of food and supplements, and infection complication.

15. The method of any one of the preceding claims, further comprising defining a suitable treatment or patient care management and/or treating the individual in accordance with the assessment of an individual's medical outcome.

16. An electronic system comprising a processor and memory containing instructions, which when executed by the processor, direct the processor to perform the method of any one of claims 1 to 14.

17. A computer program comprising software instructions which, when executed by a processor, carry out the method of any one of claims 1 to 14.

FIG.1

104 — **b. Multi-omic and clinical data collection**

Single-cell and plasma proteomics

106 — **c. Predictive modeling of medical outcome**

High risk

Mulit-Omic Bootstrap (MOB) machine learning algorithm

Low risk

102 — **a. Patient sample collection**

108 — **d. Optimization of medical decision**

Risk assessment guides medical decision and improves outcomes

Pre-event    Event    Post-event

FIG.2 (Beginning)

Original cohort

202

Sampling from original cohort

Sampling 1

Sampling 2

Sampling n

204

| Model 1 |
|---|
| Var 1 |
| Var 3 |
| Var 21 |
| Artificial 6 |

| Model 2 |
|---|
| Var 2 |
| Var 3 |
| Artificial 21 |
| Var 8 |

| Model n |
|---|
| Var 1 |
| Artificial 5 |
| Artificial 25 |
| Var 6 |

EP 4 343 775 A1

FIG.2 (Continued)

EP 4 343 775 A1

FIG.2 (End)

FIG.3

FIG.4

FIG.5

## FIG.6

EP 4 343 775 A1

FIG.7

Europäisches
Patentamt

European
Patent Office

Office européen
des brevets

# EUROPEAN SEARCH REPORT

**Application Number**

EP 22 30 6403

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/118559 A1 (LEFKOFSKY ERIC [US]) 22 April 2021 (2021-04-22) * paragraph [0049] * * paragraph [0079] - paragraph [0080] * * paragraph [0093] * * paragraph [0094] * * paragraph [0173] * * paragraph [0180] * * paragraph [0183] * * paragraph [0178] * * figures 2,3,7,8 * * paragraph [0182] * * paragraph [0211] - paragraph [0213] * ----- | 1-17 | INV. G16H20/00 G16H50/20 G16H50/50 G16H50/70 |
| X | WO 2018/220600 A1 (UNIV FLORIDA [US]) 6 December 2018 (2018-12-06) * paragraph [0010] - paragraph [0014] * * paragraph [0029] - paragraph [0031] * * paragraph [0038] * * paragraph [0059] - paragraph [0061] * * paragraph [0067] * ----- | 1-17 | |
| X | US 2019/122330 A1 (SAGET EDOUARD [US] ET AL) 25 April 2019 (2019-04-25) * paragraph [0090] - paragraph [0092] * * paragraph [0093] - paragraph [0097] * * paragraph [0130] - paragraph [0133] * * paragraph [0138] - paragraph [0142] * * paragraph [0152] - paragraph [0157] * ----- | 1-17 | TECHNICAL FIELDS SEARCHED (IPC)  G16H |

The present search report has been drawn up for all claims

| Place of search | Date of completion of the search | Examiner |
|---|---|---|
| Munich | 22 February 2023 | Megalou-Nash, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

3

## ANNEX TO THE EUROPEAN SEARCH REPORT
## ON EUROPEAN PATENT APPLICATION NO.

EP 22 30 6403

This annex lists the patent family members relating to the patent documents cited in the above-mentioned European search report.
The members are as contained in the European Patent Office EDP file on
The European Patent Office is in no way liable for these particulars which are merely given for the purpose of information.

22-02-2023

| Patent document cited in search report | Publication date | Patent family member(s) | Publication date |
|---|---|---|---|
| US 2021118559 A1 | 22-04-2021 | NONE | |
| WO 2018220600 A1 | 06-12-2018 | US 2020161000 A1 | 21-05-2020 |
| | | WO 2018220600 A1 | 06-12-2018 |
| US 2019122330 A1 | 25-04-2019 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7018850 B
- US 20120178183 A
- WO 9954494 A
- US 20010006787 A
- US 61048657

### Non-patent literature cited in the description

- **HEALY MA et al.** *JAMA Surg,* 2016, vol. 151 (9), 823-30
- **EAMER G et al.** *Am J Surg,* 2018, vol. 216 (3), 585-594
- **COHEN ME et al.** *J Am Coll Surg,* 2017, vol. 224 (5), 787-795
- **STOECKLEIN VM et al.** *J Leukoc Biol,* 2012
- **GAUDILLIERE B et al.** *Sci Trans! Med,* 2014, vol. 6 (255), 255ra131
- **TIBSHIRANI, ROBERT.** Regression shrinkage and selection via the lasso. *Journal of the Royal Statistical Society: Series B (Methodological),* 1996, vol. 58.1, 267-288
- **ZOU ; HUI ; TREVOR HASTIE.** Regularization and variable selection via the elastic net. *Journal of the royal statistical society: series B (statistical methodology),* 2005, vol. 67.2, 301-320
- **KRISTEN K. RUMER et al.** Integrated single-cell and plasma proteomic modeling to predict surgical site complications, a prospective cohort study. *Ann Surg.,* 2022, vol. 275 (3), 582-590
- **VAN HEST et al.** *FEBS Lett,* 22 May 1998, vol. 428 (I-2), 68-70
- **TANG et al.** *Abstr. Pap Am. Chem.,* 22 August 1999, vol. 2, S218
- **CHATTOPADHYAY et al.** *Nat. Med.,* 2006, vol. 12, 972-977
- **ERKKI et al.** *J. Histochemistry Cytochemistry,* 1988, vol. 36, 1449-1451
- **TANNER et al.** *Spectrochimica Acta Part B: Atomic Spectroscopy,* 2007, vol. 62 (3), 188-195
- **LAKOWICZ, J. R.** Principles of Fluorescence Spectroscopy. Plenum Press, 1983
- Resonance energy transfer microscopy, in: Fluorescence Microscopy of Living Cells in Culture. **HERMAN, B.** Methods in Cell Biology. Academic Press, 1989, vol. 30, 219-243
- **TURRO, N. J.** Modern Molecular Photochemistry. Benjamin/Cummings Publishing Col, Inc, 1978, 296-361
- **SHAPIRO, HOWARD M.** Practical Flow Cytometry. John Wiley & Sons, Inc, 2003

- **TANNER et al.** Spectrochimica Acta. *Atomic Spectroscopy,* March 2007, vol. 62 (3), 188-195
- **BODENMILLER.** *Nature Biotechnology,* 2012, vol. 30, 858-867
- **ALBERTS et al.** The Molecular Biology of the Cell. Garland Science, 2002
- **VOGELSTEIN ; KINZLER.** The Genetic Basis of Human Cancer. McGraw Hill, 2002
- **MICHAEL.** Biochemical Pathways. John Wiley and Sons, 1999
- **WEINBERG.** *The Biology of Cancer,* 2007
- **JANEWAY et al.** Immunobiology. Garland
- Growth Factors and Cytokines in Health and Disease. **LEROITH ; BONDY.** A Multi Volume Treatise. Growth Factors, 1996, vol. 1A
- **SAMBROOK et al.** Molecular Cloning: A Laboratory Manual. Harbor Laboratory Press, 2001
- Short Protocols in Molecular Biology. John Wiley & Sons, 1999
- **BOLLAG et al.** Protein Methods. John Wiley & Sons, 1996
- Nonviral Vectors for Gene Therapy. Academic Press, 1999
- Viral Vectors. Academic Press, 1995
- Immunology Methods Manual. Academic Press, 1997
- **DOYLE ; GRIFFITHS.** Cell and Tissue Culture: Laboratory Procedures in Biotechnology. John Wiley & Sons, 1998
- **NICOLAI MEINSHAUSEN ; PETER BÜHLMANN.** *Ann. Statist.,* June 2006, vol. 34 (3), 1436-1462
- **CANDES ; EMMANUEL et al.** Panning for gold:'model-X'knockoffs for high dimensional controlled variable selection. *Journal of the Royal Statistical Society: Series B (Statistical Methodology),* 2018, vol. 80.3, 551-577
- **BACH ; FRANCIS R.** Bolasso: model consistent lasso estimation through the bootstrap. *Proceedings of the 25th international conference on Machine learning,* 2008

- **MEINSHAUSEN, NICOLAI.** Relaxed lasso. *Computational Statistics & Data Analysis,* 2007, vol. 52.1, 374-393
- **WANG, SIJIAN et al.** Random lasso. *The annals of applied statistics,* 2011, vol. 5.1, 468

- Applications of the lasso and grouped lasso to the estimation of sparse graphical models. **FRIEDMAN ; JEROME ; TREVOR HASTIE ; ROBERT TIBSHIRANI.** Technical report. Stanford University, 2010
- **EYRAUD ; REMI ; COLIN DE LA HIGUERA ; JEAN-CHRISTOPHE JANODET.** LARS: A learning algorithm for rewriting systems. *Machine Learning,* 2007, vol. 66.1, 7-31